(19) Europäisches Patentamt
European Patent Office
Office européen des brevets

(11) **EP 4 765 135 A1**

(12) **EUROPEAN PATENT APPLICATION**

(43) Date of publication:
**24.06.2026 Bulletin 2026/26**

(21) Application number: **24221535.8**

(22) Date of filing: **19.12.2024**

(51) International Patent Classification (IPC):
**G16H 10/40** (2018.01)　　**G01N 35/00** (2006.01)
**G16H 40/40** (2018.01)

(52) Cooperative Patent Classification (CPC):
**G16H 10/40; G01N 35/00594; G01N 35/00722;**
**G01N 35/0092; G16H 40/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **SYSMEX CORPORATION
Kobe-shi
Hyogo 651-0073 (JP)**

(72) Inventors:
• **NAGAO, Yuto
Kobe-shi, Hyogo, 651-0073 (JP)**
• **UEMATSU, Sakiko
Kobe-shi, Hyogo, 651-0073 (JP)**
• **BAUER, Gabi
Kobe-shi, Hyogo, 651-0073 (JP)**
• **HEIDINGER, Maros
Kobe-shi, Hyogo, 651-0073 (JP)**

(74) Representative: **Hoffmann Eitle
Patent- und Rechtsanwälte PartmbB
Arabellastraße 30
81925 München (DE)**

(54) **SYSTEM FOR EVALUATION OF INTER-INSTRUMENT DIFFERENCE**

(57)　Disclosed is a sample analysis system comprising: a plurality of measurement units, each configured to measure a sample to obtain a measurement result based on a measurement order including one or more measurement items; an identification information reader configured to read identification information of each of a plurality of first samples to be measured for diagnosis of a test subject by at least one of the plurality of measurement units; and a computer that is configured to: identify at least one of the plurality of first samples as a second sample used for a measurement for an inter-instrument difference evaluation, based on a timing to conduct the inter-instrument difference evaluation, wherein the timing is automatically determined by the computer; and control each of a plurality of target measurement units to measure the identified second sample based on a measurement order including one or more preset measurement items for the inter-instrument difference evaluation, and store a plurality of second measurement results of the second sample obtained from the plurality of target measurement units, for use in the inter-instrument difference evaluation, in the storage device in association with the identification information of the second sample.

Fig. 5A

## Description

## Technical Field

The present disclosure relates to a sample analysis system and corresponding computer program. In particular, the present disclosure relates to a sample analysis system and corresponding computer program for evaluating an inter-instrument difference, that is, evaluating measurement differences among measurement units measuring a same sample.

## Background

If more than one sample measurement unit is installed at a facility, it may be required to be able to obtain equivalent measurement results by each of the sample measurement units when the same sample is measured by each of the sample measurement units for a same measurement item. In facilities where multiple sample measurement units are installed, in order to confirm the equivalence of measurement results by each sample measurement unit (inter-instrument difference evaluation) on a regular basis, the same sample is measured by each of target sample measurement units that are subject to the inter-instrument difference evaluation and measurement results are compared among the target sample measurement units.

## Summary

## Technical Problem

Conventionally, for example according to S. Park, et al. (December, 2014). Am J Clin Pathol 2014;142:777-787, Development and Validation of Effective Real-Time and Periodic Interinstrument Comparison Method for Automatic Hematology Analyzers, when performing the inter-instrument difference evaluation, operator involvement has been significantly required. Hence, there is a problem that a huge burden is placed on the operator. There is thus a need to reduce the burden of the operator when performing the inter-instrument difference evaluation.

## Solution

According to an aspect of the present disclosure, a sample analysis system comprises: a plurality of measurement units, each configured to measure a sample to obtain a measurement result based on a measurement order including one or more measurement items; an identification information reader configured to read identification information of each of a plurality of first samples to be measured for diagnosis of a test subject by at least one of the plurality of measurement units; and a computer that communicates with the identification information reader and the plurality of measurement units. The first sample is measured by at least one of the plurality of measurement units based on a first measurement order for the diagnosis of the test subject, and a first measurement result of the first sample obtained from the at least one of the plurality of measurement units is stored, for use in the diagnosis of the test subject, in a storage device in association with the identification information of the first sample. A plurality of target measurement units designated from the plurality of measurement units are subject for inter-instrument difference evaluation. The computer is configured to: identify at least one of the plurality of first samples as a second sample used for the measurement for the inter-instrument difference evaluation, based on a timing to conduct the inter-instrument difference evaluation, wherein the timing is automatically determined by the computer; and control each of the plurality of target measurement units to measure the identified second sample based on a second measurement order including one or more preset measurement items for the inter-instrument difference evaluation, and store a plurality of second measurement results of the second sample obtained from the plurality of target measurement units, for use in the inter-instrument difference evaluation, in the storage device in association with the identification information of the second sample.

According to another aspect of the present disclosure, a computer program product comprises instructions which cause a computer (110) to carry out operations for inter-instrument difference evaluation, wherein the computer is in communication with: a plurality of measurement units (160), each configured to measure a sample to obtain a measurement result based on a measurement order including one or more measurement items; and an identification information reader configured to read identification information of each of a plurality of first samples to be measured for diagnosis of a test subject by at least one of the plurality of measurement units. The first sample is measured by at least one of the plurality of measurement units based on a first measurement order for the diagnosis of the test subject, and a first measurement result of the first sample obtained from the at least one of the plurality of measurement units is stored, for use in the diagnosis of the test subject, in a storage device in association with the identification information of the first sample. A plurality of target measurement units designated from the plurality of measurement units are subject for inter-instrument difference evaluation. The operations for the inter-instrument difference evaluation comprise: identifying at least one of the plurality of first samples as a second sample used for the measurement for the inter-instrument difference evaluation, based on a timing to conduct the inter-instrument difference evaluation, wherein the timing is automatically determined by the computer; and controlling each of the plurality of target measurement units to measure the identified second sample based on a second measurement order including one or more preset measurement items for the inter-instrument difference evaluation, and storing a plurality of second measurement results of the second sample obtained from the plurality of target measurement units, for use in the inter-instrument difference evaluation, in the storage device in association with the identification information of the second sample.

## Brief Description of Drawings

**Fig. 1** shows an example of a sample analysis system.

**Fig. 2** shows an example of a computer of the sample analysis system.

**Fig. 3** shows an example of an analyzer of the sample analysis system.

**Fig. 4** shows an example of a data analysis unit and a measurement unit of an analyzer of the sample analysis system.

**Fig. 5A** shows an example process to store multiple measurement results of a target sample for evaluation of an inter-instrument difference.

**Fig. 5B** shows an example for identifying a target sample and measurements of the target sample by each of a plurality of target measurement units.

**Fig. 5C** shows an example graphical user interface for setting inter-instrument difference evaluation.

**Fig. 6A** shows an example data structure in a database for storing measurement results.

**Fig. 6B** shows an example process to generate evaluation information for the evaluation of inter-instrument difference.

**Fig. 6C** shows an example data structure in a database for storing measurement results.

**Fig. 7** shows an example of the evaluation information.

**Fig. 8** shows another example data structure in a database for storing measurement results.

**Fig. 9** shows another example data structure in a database for storing measurement results.

**Fig. 10** shows another example process to generate evaluation information.

**Fig. 11** shows an example of using a data analysis unit of an analyzer as a computer.

**Fig. 12** shows another example of a sample analysis system.

**Fig. 13** shows an example process to generate evaluation information.

**Fig. 14** shows an example graphical user interface for setting a timing to conduct a measurement for inter-instrument difference evaluation.

**Fig. 15** shows an example graphical user interface for setting a number of target samples.

**Fig. 16** shows another example process to generate evaluation information.

**Fig. 17** shows another example of the evaluation information.

**Fig. 18** shows another example of a sample analysis system.

**Fig. 19** shows an example grouping of a master device with respective measurement units.

**Fig. 20** shows an example of a unique identifier for a target sample.

**Fig. 21** shows an example process of a sample analysis system using the unique identifier.

**Fig. 22** shows an example data structure for storing measurement results in association with sample IDs.

**Fig. 23** shows an example of a unique identifier for a target sample.

**Fig. 24** shows an example process of a sample analysis system using the unique identifier.

**Fig. 25** shows an example data structure for storing measurement results in association with rack IDs.

**Fig. 26** shows another example of a sample analysis system.

**Fig. 27** shows another example of a sample analysis system.

**Fig. 28** shows an example storage device including a database for storing measurement results and selection criteria.

**Fig. 29** shows an example criteria table of selection criteria used for selecting a sample.

**Fig. 30** shows an example of a graphical user interface for setting and combining parameters for setting selection criteria.

**Fig. 31** shows an example of a graphical user interface for setting message parameters for usage as selection criteria.

**Fig. 32** shows an example of a graphical user interface for setting flag parameters for usage as selection criteria.

**Fig. 33** shows a list of selected target samples.

**Fig. 34** shows an example process to generate and output a report related to an evaluation of inter-instrument difference.

**Fig. 35** shows an example report for inter-instrument difference evaluation.

**Detailed Description**

The present invention is related to a sample analysis system for simplifying the inter-instrument difference evaluation. Inter-instrument difference evaluation is to evaluate difference among measurement results obtained from a plurality of different measurement units when each of the measurement units measures a same sample (a target sample). In a situation where multiple measurement units may be used in an operator's site such as a hospital or a laboratory, the inter-instrument difference evaluation is used to ensure accurate and compatible measurements among the multiple measurement units. The inter-instrument difference evaluation may also be used to initiate or trigger an appropriate action if the inter-instrument difference evaluation shows that a measurement result of at least one measurement unit deviates from those of other measurement unit(s) beyond an allowable range. For example, the appropriate action may be an initiation of a cleaning operation, a suspension of the suspicious measurement unit (and a corresponding re-routing of samples to other measurement unit), and the like.

**Embodiment 1**

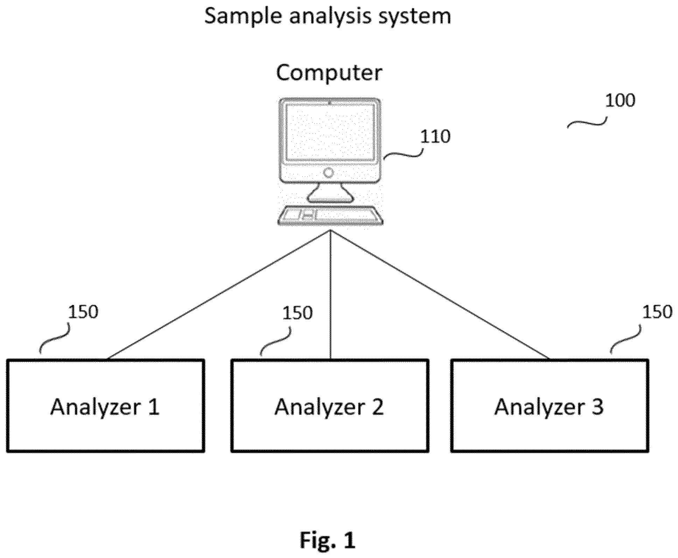

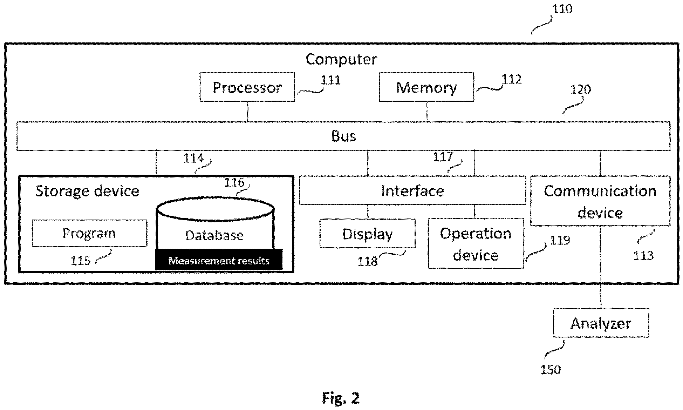

As shown in **Figs. 1** - **4,** a sample analysis system 100 according to the present disclosure includes a computation device 110 and multiple analyzers 150. While **Fig. 1** shows three analyzers 150 (analyzers 1 - 3), this is a non-limiting example of a sample analysis system. A computation device 110 (referred to as computer below, for brevity) may be a machine capable of performing calculation and processing data, and may be a personal computer, an embedded computation device, a mobile device such as a smart phone and a tablet, a server or the like. The sample analysis system 100 may be arranged in an operator's site. Alternatively, the analyzers 150 may be arranged in the operator's site and may be connected with the computer 110 remotely accessible from the operator's site. For example, the computer 110 remotely accessible from the operator's site may be a computer in a cloud infrastructure. As shown in **Fig. 2,** the computer 110 may include a processor 111, a memory 112, a communication device 113, a storage device 114, an interface 117 connecting a display 118 and/or an operation device 119 to a communication bus 120. The storage device 114 may include a program 115 for executing the inter-instrument difference evaluation as described below, and a database 116 for storing measurement results obtained by at least one of the analyzers 150. While **Fig. 2** shows, for illustrative purposes, a communication line (which may be a wired or wireless communication line) between the communication device 113 of the computer 110 and an analyzer 150, the skilled person understands that the communication device 113 may be communicatively connected with more than one analyzer 150 and may suitably be configured to receive and transmit data simultaneously or sequentially to these analyzers 150.

Each of the multiple analyzers 150 may be configured to perform a measurement on a biological sample. The measurement result may be utilized by doctors or clinical laboratory operators. For example, based on the measurement result, the doctor may diagnose a patient and determine a medical treatment. For example, the analyzer 150 may be a hematology analyzer (blood cell analyzer), a urine analyzer, a coagulation analyzer, etc. The operator's site may have various types of analyzers. For example, the operator's site may have a plurality of hematology analyzers and a plurality of urine analyzers. However, the inter-instrument difference is evaluated among a same type of analyzers (i.e., the inter-instrument difference among hematology analyzers is evaluated only within a group of hematology analyzers). The same type of analyzers may refer to analyzers that measure samples using a same measurement principle and same type of reagents.

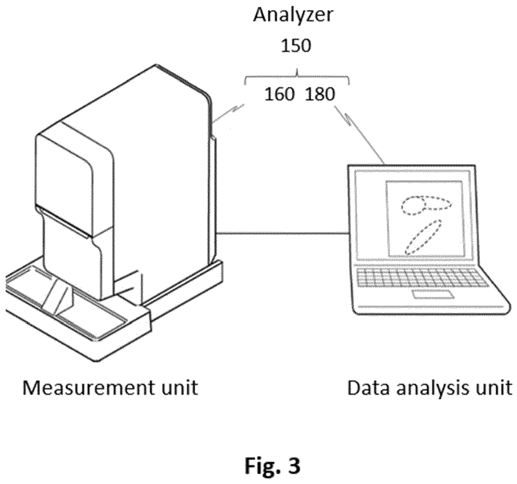

As shown in **Fig. 3,** each analyzer 150 may include a measurement unit 160 and a data analysis unit 180. While **Fig. 3** shows the measurement unit 160 and the data analysis unit 180 as being two different hardware entities, the skilled person understands that the measurement unit 160 and the data analysis unit 180 may also be embedded or integrated into a single entity. The measurement unit 160 may measure a biological sample and may transmit measurement data to the data analysis unit 180 which may process the measurement data to generate measurement result. The data analysis unit 180 may be connected to the computer 110, as shown in **Fig. 1,** and the generated measurement result may be transmitted to the computer 110 from the data analysis unit 180. The multiple measurement units 160 in the sample analysis system may be configured to perform optical and/or electrical measurements. The present disclosure may be applied to any sample analysis system in which a measurement for one or more measurement items of the sample are commonly performed among the multiple measurement units. For example, the multiple measurement units may be a same type of measurement units that perform a measurement of blood cells using flow-cytometry for one or more measurement items that are common among the measurement units. For example, the multiple measurement units may be a same type of measurement units that perform morphological analysis for digital morphological image.

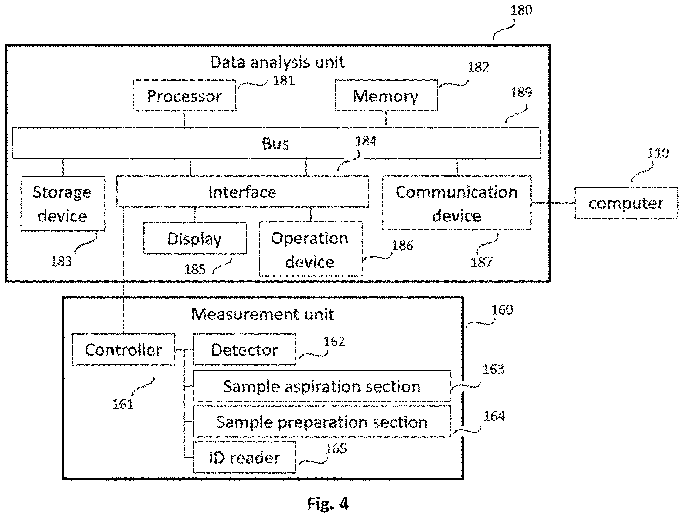

**Fig. 4** shows an example of a configuration of the analyzer 150. The analyzer 150 may have the data analysis unit 180 and the measurement unit 160, as described in **Fig. 3**. As discussed, the data analysis unit 180 and the measurement unit 160 may be embedded in a same entity or may be associated with two different entities. The data analysis unit 180 may include a processor 181, a memory 182, a storage device 183, an interface 184 for a display 185 and an operation device 186, and a communication device 187. The processor 181, the memory 182, the storage device 183, the interface 184, and the communication device 187 interact via a bus 189. The measurement unit 160 may be configured with a controller 161 that interacts, preferably based on instructions received from the data analysis unit 180, with other parts including, for example one or more detectors 162, a sample aspiration section 163, a sample preparation section 164, an identification information (ID) reader 165, such as a barcode reader, or the like. The sample aspiration section 163 aspirates a sample from a sample container into the measurement unit 160 through a sample nozzle. The sample preparation section 164 prepares a measurement sample by mixing a portion (aliquot) of the aspirated sample and at least one reagent such as a diluting solution, a lysing solution or a staining solution in a chamber. As discussed, the one or more detectors 162 perform measurements on the measurement sample to acquire measurement data which may be transferred to the data analysis unit 180 via the controller 161. The data analysis unit 180 may then generate measurement results from the measurement data. These measurement results may also be displayed, manipulated, edited, filtered, or otherwise processed through interaction via the human-machine interface, and subsequently sent to the computer 110 along with associated sample information such as sample identification information.

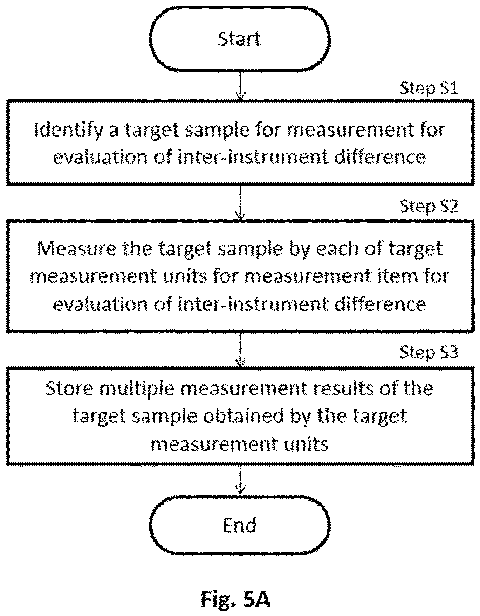

**Fig. 5A** shows a process to store multiple measurement results of the target sample. The process may be performed by the computer 110 of **Fig. 1,** in particular by the computer 110 equipped with the processor 111 executing corresponding instructions as defined in the computer program 115. In step **S1** of **Fig. 5A,** the computer 110 may automatically identify the target sample from a plurality of samples to be measured. The target sample is to be used for a measurement for inter-instrument difference evaluation. The sample other than the target sample is to be used for a measurement to acquire the measurement result for diagnosing and determining the medical treatment. The computer 110 may identify, from a plurality of first samples to be measured for diagnosing and determining the medical treatment, a second sample (the target sample) used for measurement for the evaluation of inter-instrument difference. The target measurement units are subject for the inter-instrument difference evaluation. The computer 110 may identify all measurement units 160 installed in the sample analysis system 100 as the target measurement units or may identify some or a subset of the measurement units 160 as the target measurement units. The target measurement units may be designated by the operator. The first samples may be used for diagnosing and determining the medical treatment. The second sample (the target sample) identified from the first samples may be used for the inter-instrument difference evaluation, and may also be used for diagnosing and determining the medical treatment. As the target sample, a biological sample such as a blood sample collected from a test subject (e.g., patient) may be used. The computer 110 may identify the target sample based on an ID associated with the sample (e.g., the ID of the first sample). Among the IDs of the first samples, at least one of the ID may be designated as the ID of the target sample. For example, the operator may designate the first sample to be used for the inter-instrument difference evaluation, and set the ID of the designated first sample into the computer 110. For example, the computer 110 may monitor the IDs of the first samples and determine if the monitored ID matches with the ID of the designated first sample. If the monitored ID matches with the ID of the designated first sample, the computer 110 identifies the first sample with the matched ID as the target sample. The skilled person understands that the target sample can be used for inter-instrument difference evaluation by evaluating a difference in measurement data or measurement results of the target sample measured by different target measurement units 160.

Instead of a sample collected from the test subject, a quality control sample which is prepared with known quantities of specific analytes may be utilized as the target sample. Conversely, in a facility that prefers the sample collected from the test subject to the quality control sample for the measurement for the inter-instrument difference evaluation, the computer 110 may perform a process to exclude the quality control sample from being identified as the target sample. For example, the computer 110 may distinguish the quality control sample from samples collected from the test subjects, based on the ID corresponding to the quality control sample. For example, the operator may set the IDs corresponding to the quality control samples into the computer 110. The computer 110 may compare these set IDs (i.e., the IDs of the quality control samples) with the IDs read from the containers of the samples to be measured by the measurement unit 160. For example, if the read ID matches with the set ID, the computer 110 may determine that the sample is the quality control sample and may not identify it as the target sample. As another example, the quality control sample may have the ID in a predetermined format which is different from an ID format of other samples. The computer 110, for example, may identify the quality control sample if the read ID has the predetermined format, and then exclude it from being identified as the target sample.

The computer 110 may identify the target sample at random. For example, the computer 110 may generate a random number each time when the computer 110 acquires the ID associated with the first sample. If the generated random number matches a predetermined number, the computer 110 may identify the corresponding sample as the target sample.

The computer 110 may identify the target sample based on a predetermined frequency. For example, the computer 110 may identify the target sample per a predetermined number of measurements. The computer 110, for example, may designate at least one sample as the target sample in every 1000 samples (e.g., the computer 110 may designate at least one sample as the target sample each time 1000 measurements are done in the sample analysis system 100). For example, the computer 110 may identify the target sample per predetermined interval (e.g., per 8 hours, per a week, etc).

The computer 110 may identify the target measurement units 160 based on a preset rule. The preset rule may be configured in the computer 110. The preset rule, for example, may be (i) predetermined group(s) of a plurality of measurement units 160, and (ii) for the predetermined group(s), predetermined timing/cycle of the inter-instrument difference evaluation. For example, the computer 110 may identify the predetermined group(s) to which the predetermined timing/cycle comes, then the computer 110 may designate the measurement units 160 of the identified group(s) as the target measurement units 160.

The measurement result of the target sample (i.e., the second sample) may be used for the inter-instrument difference evaluation, and also be used for the diagnosis of the test subject (i.e., patient). That is, the measurement result of the target sample may serve both the patient diagnosis and the inter-instrument difference evaluation. For example, if one or more measurement items used in the inter-instrument difference evaluation cover measurement items required for diagnosis of the test subject, the measurement of the target sample for diagnosis of the test subject may be omitted.

In step **S2** of **Fig. 5A,** each of the target measurement units 160 may conduct a same measurement of the target sample for one or more measurement items (e.g., measurement parameters) for the evaluation of inter-instrument difference. In other words, each of the plurality of target measurement units 160 may measure the second sample based on a measurement order including one or more preset measurement items for the inter-instrument difference evaluation, with these measurement items being common or the same among the target measurement units 160.

The computer 110 may generate the measurement order including the one or more measurement items which have been set for the measurement for the inter-instrument difference evaluation and transmit the generated measurement order to each of the target measurement units 160. As will also be further detailed below, the measurement items may be set by the operator, for example, via a graphical user interface. In one exemplified case where the measurement units M1 - M3 of corresponding analyzers 1 - 3 in **Fig. 1** are the target measurement units 160, the computer 110 may send the measurement order to each of the measurement units M1 - M3 to measure the target sample for the same measurement items for the evaluation of inter-instrument difference.

After the measurement by each of the target measurement units M1 - M3, measurement results may be sent to the computer 110, and in step **S3** of **Fig. 5A,** the measurement results of the target sample may be stored in the storage device 114 (database 116) of the computer 110 in association with ID of the target sample for use in the inter-instrument difference evaluation. In other words, the computer 110 may store a plurality of measurement results of the second sample obtained by measurements by the plurality of measurement units 160 in the storage device 114 for use in the inter-instrument difference evaluation.

In step **S1** of **Fig. 5A,** the computer 110 may identify a plurality of second samples (two or more target samples) from the plurality of first samples, and in step **S3,** the computer 110 may, for each of the identified second samples, store the plurality of measurement results of the second sample in the storage device for use in the inter-instrument difference evaluation. Here, the IDs for each of the identified second samples are mutually different from each other.

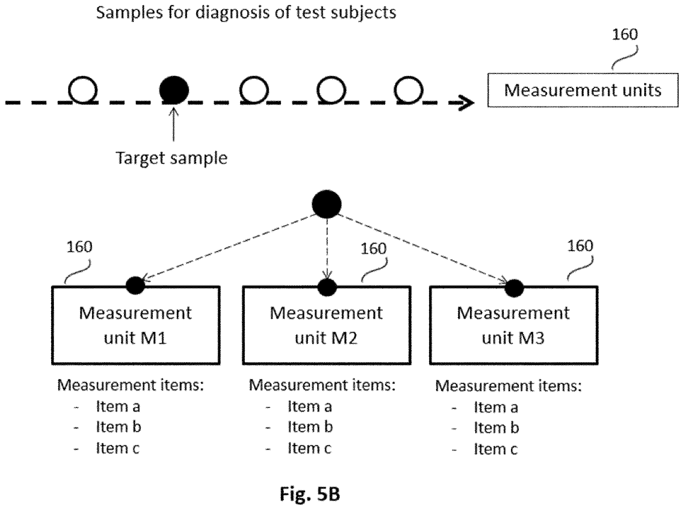

**Fig. 5B** illustrates an exemplary concept of identifying the target sample and performing measurement of the target sample by each of the target measurement units 160. The sample analysis system 100 routinely receives a plurality of samples (i.e., the first samples) which are to be measured by at least one of the measurement units 160. These samples (i.e., the first samples) may be used for measurement for diagnosis of the test subjects such as patients. When the computer 110 determines that a timing to conduct the inter-instrument difference evaluation has arrived, at least one sample (i.e., the first sample) may be automatically identified as the target sample (i.e., the second sample) by the computer 110 and the target sample may be automatically measured by each of the target measurement units 160 for the inter-instrument difference evaluation. The target sample may be aspirated using the sample aspiration section 164 by each of the target measurement units 160 from the sample container which contains the target sample, that is, a portion of the same target sample may be aspirated by each of the target measurement units 160, and the aspirated sample is measured by the detector(s) 162 for one or more (same or common) measurement items by each of the target measurement units 160.

The one or more measurement items include at least one measurement item that is common among the target measurement units 160. All measurement items may be common among the target measurement units 160, or measurement items may be partially overlapped among the target measurement units 160. For example, as illustrated in **Fig. 5B,** each of the measurement units M1, M2, M3 may measure the target sample for the same measurement items a, b, c. The skilled person understands that the inter-instrument difference may then be evaluated by comparing measurement results obtained from these measurements.

The plurality of the target measurement units 160 may comprise at least a first measurement unit and a second measurement unit. Both of the first and second measurement units may be designated as the target measurement units, and measure the second sample which is designated as the target sample. Here, the first measurement unit may aspirate a portion of the second sample, which is the same sample measured/to be measured by the second measurement unit, from a sample container containing the second sample and measure the aspirated second sample for the one or more measurement items for the inter-instrument difference evaluation, and the second measurement unit may aspirate a portion of the second sample, which is the same sample measured/to be measured by the first measurement unit, from the sample container and measure the aspirated second sample for the one or more measurement items for the inter-instrument difference evaluation.

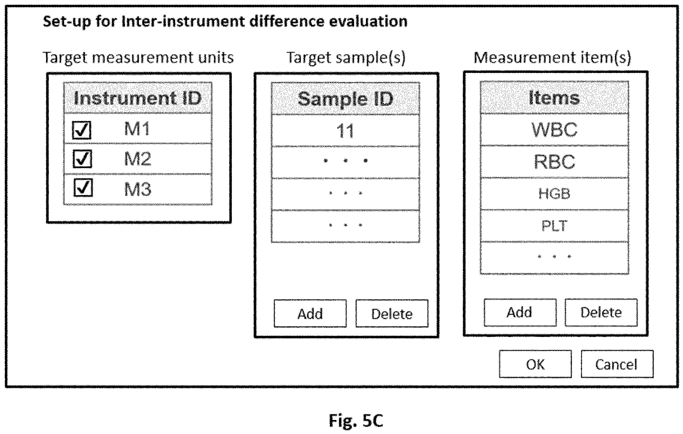

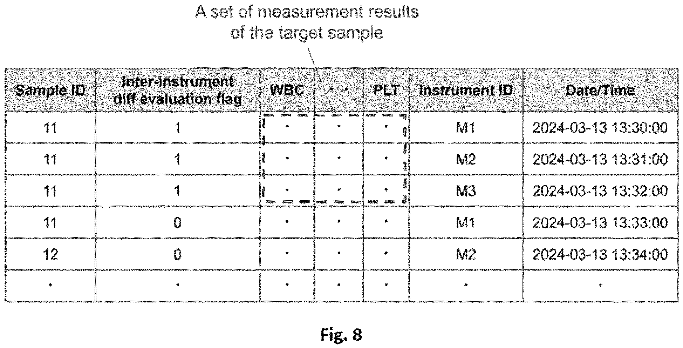

In one exemplified case, as shown in **Fig. 5C,** the computer 110 may receive operator's designation of the target measurement units, target samples and one or more measurement items for the inter-instrument difference evaluation. If the computer 110 has received a designation of the target sample, the computer 110 may store, in the database 116 as shown in **Fig. 8,** flag information indicating the inter-instrument difference evaluation in association with the ID (as shown "11" in Fig. 8) of the designated target sample. If the computer 110 autonomously designates the target sample, the computer 110 may store, in the database 116 as shown in the Fig.8, the flag information in association with the ID of the designated target sample. When a plurality of sample containers are supplied one by one to at least one of the measurement units 160 by the operator or a sample transport device, the ID attached to each supplied sample container may be read by ID reader (identification information reader) of the sample analysis system. For example, the ID reader 165 such as a barcode reader, located at each measurement unit 160 may be utilized for reading the ID. The ID read by the ID reader may then be transmitted to the computer 110. Along with the ID of the sample, the ID of the measurement unit 160 (Instrument ID), which measures the sample corresponding to the ID of the sample, may also be transmitted to the computer 110. For example, when the ID of the sample is read by the ID reader 165, the measurement unit 160 may transmit both the ID of the sample and the Instrument ID to the computer 110. The computer 110 may determine whether the sample is the target sample to be measured by the target measurement unit based on comparison of the ID read by the ID reader and the ID of the designated target sample. The computer 110 may compare the Instrument ID sent from the measurement unit 160 and the designated Instrument ID as shown in **Fig. 5C,** and, if these IDs are matched (i.e., the measurement unit is recognized as the target measurement unit), the computer 110 may send the measurement order for the inter-instrument difference evaluation to the measurement unit 160. In other words, the identification information reader of the sample analysis system 100 may read IDs of first samples, and the computer 110 may identify the second sample (target sample) from a plurality of the first samples whose IDs have been read by the identification information reader. When the computer 110 has identified the target sample, the computer 110 may send the measurement order to instruct the target measurement unit 160 to measure the target sample for one or more measurement items which have been set for the inter-instrument difference evaluation. As illustrated in **Fig. 5C,** setting for inter-instrument difference evaluation may be achieved via a graphical user interface, for example, at the computer 110, allowing to select, for one or more sample IDs (which may be added or deleted), the target measurement units 160 to be subjected for the inter-instrument difference evaluation and the one or more measurement items to be measured at the respective target measurement units 160. Here, in particular, the target sample with sample ID "11" should be used to determine measurement data of the measurement items related to white blood counts (WBC), red blood counts (RBC), hemoglobin (HGB) and platelets (PLT) on each of the measurement units 160 having Instrument ID M1, M2 and M3, for the purpose of the inter-instrument difference evaluation. Here, the measurement units 160 may be blood cell counting units for counting respective blood cells (WBC, RBC, PLT etc.) in a blood sample. Consequently, blood cell counts for the identical target sample can be determined at each of M1, M2, M3 and these counts may be used to determine and evaluate an inter-instrument difference. The skilled person understands that additional or fewer instrument IDs, sample IDs, and/or measurement items can be selected, for example via the user interface.

In a case where a plurality of sample containers are transported to at least one of the measurement units 160 by the sample transport device, the ID reader may be located at a transport start position of the sample transport device to read the IDs from the sample containers at the transport start position. In this case, the computer 110 may identify the target sample at the transport start position based on the IDs read by the ID reader and control the transport device to transport the identified target sample to each of the target measurement units 160 without the need of the operator to manually transport the target sample to each of the target measurement units 160.

According to an aspect of the present disclosure, especially represented by the processes of **Figs. 5A-5C,** the computer 110 automatically identifies the target sample used for measurement for the inter-instrument difference evaluation from a plurality of samples to be measured by at least one of the measurement units 160. Therefore, when the target sample is included among a plurality of samples routinely tested by at least one of the measurement units 160, the target sample can be automatically identified from the plurality of samples without operator intervention during the routine testing, and each of the target measurement units 160 can be instructed to measure the target sample for the inter-instrument difference evaluation. This reduces the operator's burden of manually instructing each of the target measurement units to perform measurements on the target sample, thereby simplifying the operations for the inter-instrument difference evaluation.

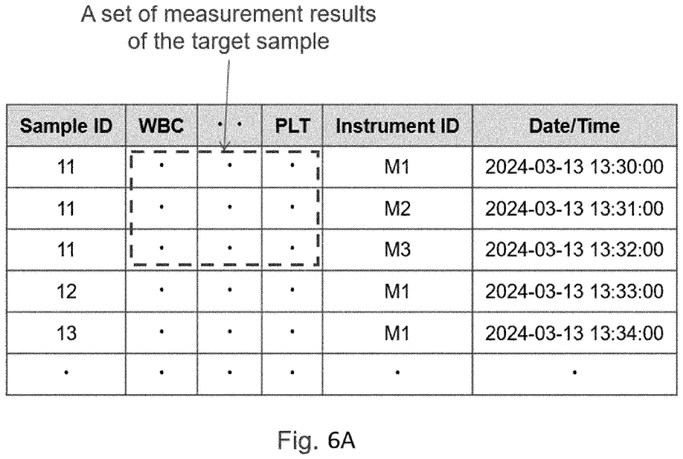

Preferably, the computer 110 stores, in the storage device 114, sample identification information of the second sample (target sample) in association with each of the plurality of measurement results of the second sample obtained by measurements by the plurality of target measurement units 160. Fig. 6A shows an exemplary data structure of the database 116. The measurement results of the target sample may be stored in this exemplary data structure which stores the measurement result (e.g., for WBC, ..., PLT) in association with the ID of the sample and the Instrument ID. The exemplary data structure may also store a date and/or time as to when the measurement has been performed.

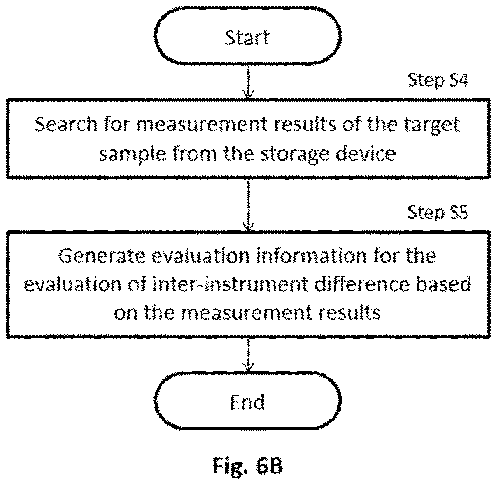

**Fig. 6B** shows a process to generate evaluation information on the inter-instrument difference based on the measurement results stored in the storage device 114. In particular, when the inter-instrument difference evaluation is conducted, the computer 110, in step **S4** of **Fig. 6B,** may search (interrogate) the database 116 for a plurality of measurement results of the target sample (second sample). The storage device 114 of the computer 110 may store in the database 116, one or more measurement results of at least one first sample which has been measured, for example, for diagnosis of a test subject, by at least one of the plurality of the measurement units 160. The storage device 114 may also store one or more sets of measurement results of at least one target sample (second sample) which has been measured, based on the measurement order including one or more preset measurement items for the inter-instrument difference evaluation, by each of the plurality of the target measurement units 160. In other words, the storage device 114 of the computer 110 may store a first measurement result of the first sample, which is obtained from measurement for diagnosis of the test subject by at least one of the plurality of the measurement units, and may also store a plurality of second measurement results of the second sample, which are obtained from measurements by the plurality of target measurement units.

The storage device 114 may also store information such as data retrieval conditions for searching for the measurement results of the target sample. The data retrieval condition may be a condition of searching for the plurality of measurement results corresponding to the identified target sample. In other words, the data retrieval condition may be for identifying a set (bundle) of measurement results for the inter-instrument difference evaluation.

In order to generate the evaluation information, the computer 110 may automatically search for the measurement results of the target sample according to the data retrieval conditions at a predetermined timing (e.g., per week, per month, per a few months, etc.). For example, in order to generate the evaluation information, the computer 110 may interrogate the storage device 114 to identify the set of the measurement results of the target sample based on the operator's instruction. For example, the operator may input search instruction to the computer 110, or may instruct the computer 110 to perform the search (interrogation) according to the data retrieval conditions.

In general, the storage device 114, in database 116, may store the measurement result of the first sample in association with first sample identification information of the first sample and store each of the measurement results of the second sample in association with second sample identification information of the second sample. The computer 110 equipped with the processor 111 may then autonomously search (interrogate), in order to generate the evaluation information, the storage device 114 for the plurality of measurement results of the second sample. Each of the plurality of measurement results of the second sample is associated with the same ID (i.e., the ID of the second sample) and is obtained by different target measurement units 160. These measurement results, which match the data retrieval conditions, may be considered as the measurement results for the inter-instrument difference evaluation, and may serve as a source of the evaluation information. In other words, in order to generate the evaluation information, the computer 110 may autonomously search for the measurement results obtained for the inter-instrument difference evaluation, and then generate the evaluation information based on the searched results. For example, regarding the exemplary data structure of **Fig. 6A,** the computer 110 may autonomously search for the measurement results (i) each corresponding to the same ID (in the Fig. 6A, the ID "11") and (ii) each corresponding to different instrument IDs (IDs "M1", "M2" and "M3").

To search for the measurement results for generating the evaluation information, the computer 110 may refer to the measurement items of the measurement results stored in the database 116. For example, the computer 110 may autonomously search for the measurement results based on the data retrieval conditions such as (i) measurement results each corresponding to the same ID, (ii) measurement results each corresponding to different measurement units, and (iii) measurement results each corresponding to the same or common measurement items (i.e., for the inter-instrument difference evaluation, each result may be required to correspond to the same or common measurement items). Regarding the above mentioned (ii), it may be substituted with a condition such as "measurement results each corresponding to the designated target measurement units". In this substitution, the computer 110 may refer to the setting (as shown in **Fig. 5C**) regarding the instrument IDs of the designated target measurement units.

Based on such data retrieval conditions, the computer 110 may be able to extract the measurement results that match the conditions and are considered as the source of the evaluation information. Based on the autonomous search (interrogation), the computer 110 may also acquire multiple sets (bundles) of measurement results corresponding to multiple target samples. For each of the sets (bundles), the computer 110 may generate the evaluation information. This way, the computer 110 may search for a set (bundle) of measurement results of the target sample by using sample ID, measurement items and instrument ID as keys. The keys may also include Date/Time information to restrict a period to search for the measurement results of the target sample. For example, based on the Date/Time information, as illustrated in **Fig. 6A,** it can be determined whether the measurement result of the ID "11" has been obtained by each of the measurement instruments M1, M2 and M3 within a certain pre-determined time window (period). This pre-determined time window may be set in order to identify a plurality of measurement results that should not be subject to the inter-instrument difference evaluation even if each of the plurality of measurement results is stored in association with (i) the same ID and (ii) different instrument ID of the target measurement unit. For example, if the database 116 is configured to store measurement results over multiple days, the same ID may be associated with different samples in a facility where the same ID may be assigned to different samples on different days. In this case, if the pre-determined time window is set as "5 hours", "10 hours", etc., it is possible to identify a plurality of measurement results which are associated with the same ID but have been obtained over different days outside the time window. That is, if the measurement results are outside the pre-determined time window, the computer 110 may exclude these measurement results from evaluating the inter-instrument difference.

The above are examples of conditions that may be used as a criterion (search information) for selecting or filtering out measurement results to be used to generate the evaluation information for the inter-instrument difference evaluation. Such data retrieval conditions may be coded in the (computer) program 115. For example, the program 115 may include codes to retrieve measurement results under the following conditions: (i) multiple measurement results each corresponding to the same ID (i.e., the ID of the sample), (ii) multiple measurement results each corresponding to different measurement units (i.e., different target measurement units), (iii) multiple measurement results corresponding to the same or common measurement items for the inter-instrument difference evaluation, and/or (iv) multiple measurement results within the pre-determined time window.

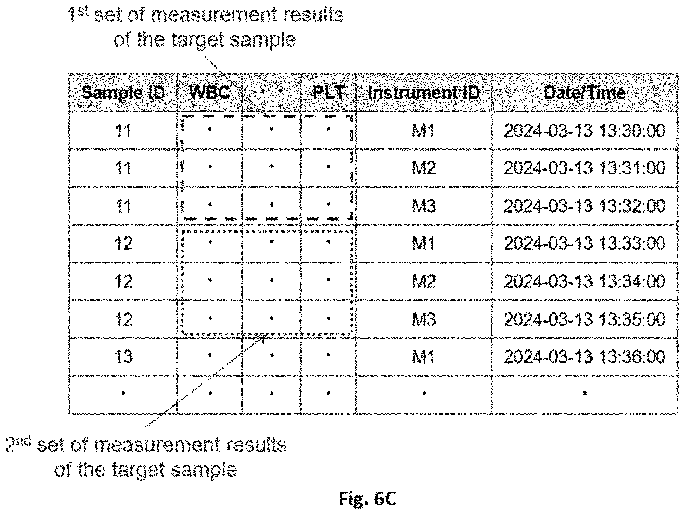

Further, in an exemplary case where different target samples (e.g., two different target samples) have been measured by each of the target measurement units M1 - M3, each of the plurality of measurement results of the target samples may be stored as a data structure in the database 116 as shown in **Fig. 6C.** In this case, the target samples have mutually different IDs (in the Fig. 6C, "11" and "12"), and measurement results of each target sample have been obtained by each of the target measurement units M1 - M3 for the same or common measurement items. The computer 110 may search for the plurality of measurement results corresponding to the ID "11" and the instrument IDs "M1", "M2" and "M3" as a first set of measurement results, and the measurement results corresponding to the (different) ID "12" and the instrument IDs "M1", "M2" and "M3" as a second set of measurement results. In other words, in this exemplary case, the computer 110 makes groups of the searched measurement results according to the ID (i.e., the ID of the target sample). In the example shown in the **Fig. 6C,** the first set of the measurement results belongs a group and the second set of the measurement results belongs another group. The computer 110 may generate the evaluation information for each of the groups.

Referring back to **Fig. 6B,** in step **S5,** the computer 110 may generate evaluation information for the inter-instrument difference evaluation based on or using the set of the measurement results of the target sample(s) (second sample) identified from the database 116 of the computer 110. In particular, the computer 110 may generate the evaluation information by comparing the identified set of measurement results to a reference or mean value, for example using statistical methods such as mean, standard deviation, coefficient of variation or acceptable bias to measure agreement or variation among target measurement units and output the evaluation information on the display.

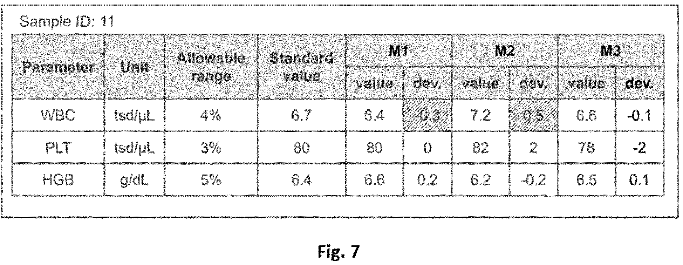

An example of the evaluation information is shown in **Fig. 7.** In this example, measurement values obtained by each of the target measurement units M1, M2, M3 for the target sample are combined in a comparable manner for each of the measured parameters (here, blood parameters: WBC, PLT, HGB, etc.), and a deviation (dev.) from a standard value for the inter-instrument difference evaluation is also included. In **Fig. 7,** a mean (average value) of the measurement values from the multiple target measurement units 160 is used as the standard value. Instead of the mean of the measurement values from the multiple target measurement units 160, the measurement value by a master device may be used as the standard value. The master device may be one of the target measurement units 160 that belong to the sample analysis system 100. The deviation from the standard value may be automatically compared with an allowable range to evaluate the inter-instrument difference. The allowable range may be an acceptable limit within which the measurement value can vary from the standard value. When the allowable range is defined as 4% of the standard value as shown in **Fig. 7,** this means that the measurement value should not deviate more than 4% above or below the standard value. When the allowable range is expressed as the percentage, the computer 110 may calculate a match rate indicating how well the measured value matches the standard value. The match rate m may be calculated based on the equation below:

$$m = \left| \frac{(y - x)}{x} \right| \times 100$$

where y is the measured value and x is the standard value. The computer 110 may compare the calculated match rate with the allowable range.

If the deviation is outside of the allowable range, a notification may be output and/or a control action may be performed. The control action may be an initiation of a cleaning operation, a suspension of operation of the measurement unit (and a corresponding re-routing of samples to other measurement units), and the like. As shown in **Fig. 7,** for example, deviations ("-0.3" for WBC in M1, which corresponds to the match rate of approximately 4.5% and "0.5" for WBC in M2, which corresponds to the match rate of approximately 7.5%) which are outside the allowable range may be displayed distinguishably from other values.

The computer 110 may receive a designation of the master device from the multiple measurement units 160. The computer 110 may receive a setting whether the mean (average value) of measurement values from the multiple target measurement units is used as the standard value or the measurement value by the master device is used as the standard value.

For example, when the computer 110 equipped with the processor 111 identifies the plurality of measurement units to be subject for the inter-instrument difference evaluation, one measurement unit selected from the identified measurement units 160 may serve and may be set as the master device which may be used for obtaining the measurement result serving as the reference for the inter-instrument difference evaluation. The master device may be selected from the measurement units 160 including the units designated for the inter-instrument difference evaluation and non-designated unit(s).

**Fig. 8** shows another example of the data structure of the database 116. In this example, the target sample used for measurement for the inter-instrument difference evaluation may be designated or selected by the operator via the operation device 119 and/or the display 118 of the computer 110. The target sample may be designated or selected by inputting a measurement order for inter-instrument difference evaluation by the operator. The computer 110 may autonomously designate the target sample. For example, the target sample may be randomly designated by the computer 110. An inter-instrument difference evaluation flag may be stored in association with the ID of the designated sample in the database. The computer 110 may then search for measurement results of the target sample by using the flag as a key. This flag can be used as search information for searching for the measurement results of the target sample. In particular, as shown in **Fig. 8,** the target sample (having the ID "11") may be associated with the inter-instrument difference evaluation flag being set (e.g. to "1") to indicate that the set of measurement results obtained for the target sample at respective different measurement units M1, M2, M3 may be used for the inter-instrument difference evaluation. On the other hand, for other samples not used as the target samples for the purpose of inter-instrument difference evaluation, the inter-instrument difference evaluation flag is not set (e.g. to "0"). Consequently, the target sample and associated set of measurement results may be readily identified and assessed by the computer 110.

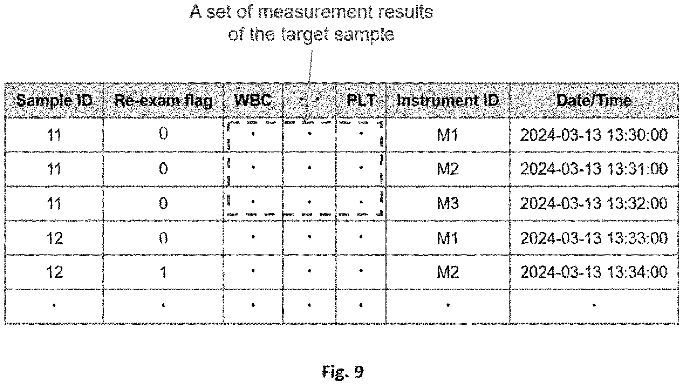

**Fig. 9** shows another example of the data structure of the database 116. Such data structure may be advantageous in a situation where, for example, an initial measurement is conducted on the first sample by the measurement unit M1 and re-measurement is conducted on the same sample by the measurement unit M2. The computer 110 may set a re-exam flag for the measurement result of the re-measurement. The re-measurement may occur for the same measurement items as the initial measurement, for example, when the measurement result obtained by the measurement unit M1 has an issue (e.g., the result has something questionable, the sample itself may have problem, the result may be unreliable, etc.). The re-measurement may also occur when the sample needs to be measured for additional measurement items that have not been measured at the initial measurement by the measurement unit M1. In such cases, although the same sample with the same ID has been measured by multiple target measurement units M1 and M2, based on the data structure, the computer 110 may advantageously be configured to not handle this sample as the target sample. The measurement result corresponding to the re-exam flag shown in the Fig. 9 is not for the inter-instrument difference evaluation but just for the re-measurement because of the issue. The computer 110 may not have to handle such sample as the target sample. Accordingly, this exemplary database 116 may be configured so that the sample which has been measured by multiple target measurement units 160 due to re-measurement can easily be distinguished by the re-exam (re-examination) flag. For example, the sample of the ID "12" measured by measurement unit M2 is associated with re-exam flag "1" which indicates that re-measurement on the sample of the ID "12" was conducted by measurement unit M2 and its measurement result is stored in the database 116 of storage device 114. In this case, the computer 110 may search for measurement results of the target sample from measurement results which are not associated with the re-exam flag "1" (or in which the re-exam flag is not set). In **Fig. 9,** measurement results of a sample of the ID "11" have been obtained by each of the target measurement units M1 - M3, and the ID "11" is not associated with re-exam flag "1" in each of the associated Instrument IDs. The computer may then readily search for measurement results of the ID "11" as measurement results of the target sample.

Instead of, or in addition to the re-exam flag, other information may be associated with the ID to exclude the sample from a scope of searching for measurement results of the target sample. For example, an error flag indicating a measurement error due to issues such as poor sensitivity of the detector 162 or deterioration of the staining solution for blood cells may be associated with the ID. This error may be detected, for example, using a hematological parameter reflecting the detector 162's sensitivity in detecting fluorescence from cells or reflecting the degree of staining of cells by the staining solution. As such parameter, for example, a median value of fluorescence intensities of all cells classified as neutrophils, which are the most common type of white blood cells, may be used. If this parameter value falls outside a preset numerical range, the measurement error may be detected. The data analysis unit 180 of the analyzer 150 may detect this error by analyzing the measurement data obtained by the measurement unit 160, and may send the error information in association with the corresponding measurement result to the computer 110. The computer 110 may search for the measurement results for the inter-instrument difference evaluation so that the measurement results associated with the error flag are excluded from the search.

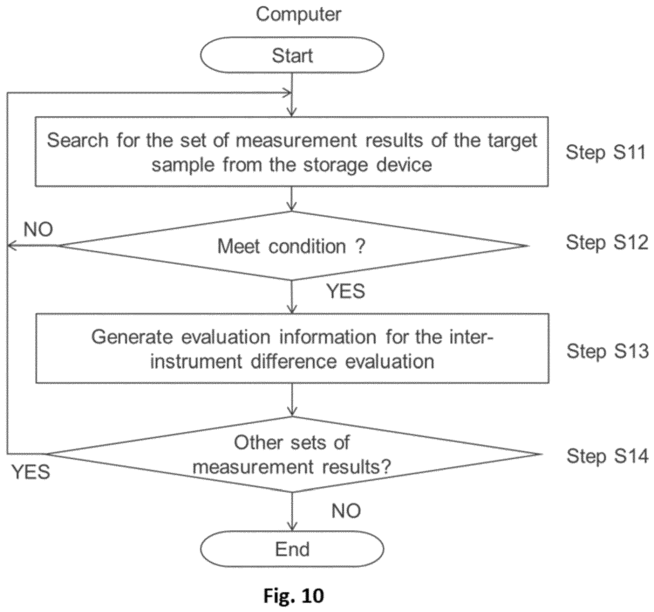

**Fig. 10** shows an exemplary process of the computer 110. In this example, in step **S11,** the computer 110 may search for a set of measurement results of the target sample from the database 116. For example, the computer 110 may search for the set of measurement results per target sample (i.e., per ID of the target sample). The computer 110 may search for the set of measurement results corresponding to one ID (e.g., the ID "11" as shown in the **Fig. 6C**). In step **S12,** the computer 110 may determine whether measurement results of the target sample meet one or more conditions for performing the inter-instrument difference evaluation. The conditions may be for identifying reliable measurement results which are suitable to be used for the inter-instrument difference evaluation. For example, the conditions may require that the measurement results of the target sample has no issues such as poor sensitivity of the detector 162, deterioration of the staining solution for blood cells, or other problems (e.g., questionable results, potential issues with the sample itself, or unreliable results, etc.). The inter-instrument difference evaluation may have to be based on measurement results obtained without these issues. According to step **S12,** the computer 110 may be able to automatically discard measurement results with such issues and avoid using them for the inter-instrument difference evaluation. If the computer 110 has determined that measurement results of the target sample meet the conditions, in step **S13,** the computer 110 may generate the evaluation information from the searched set of measurement results corresponding to the ID in the same way as in step **S5** of **Fig. 6B.** If the computer 110 has determined that measurement results of the target sample do not meet the conditions in step **S12,** the computer 110 may, in step **S14,** search for another set of measurement results corresponding to a sample ID different from the ID which has been already searched. According to step **S14,** the computer 110 may repeat to search for the set of the measurement results until there is no remaining set of results for which the evaluation information has not been generated.

According to an exemplary aspect of the present invention, especially represented by the processes of **Figs. 6A** to **10,** the computer 110 autonomously searches for measurement results of the target sample from the database which stores measurement results of the target sample as well as measurement results of samples which have been measured for diagnosis of test subjects in routine sample measurements, and autonomously generates the evaluation information based on the measurement results of the target sample which have been searched from the database. That is, the computer 110 interrogates the database to identify the set of the measurement results of the target sample, which match conditions for use in the inter-instrument difference evaluation, and generates the evaluation information based on the identified set of the measurement results. Since the computer 110 is able to autonomously perform operations for generating the inter-instrument difference evaluation, it is possible to reduce operator's workload for the inter-instrument difference evaluation.

In the above-explained embodiment, each analyzer 150 includes the measurement unit 160 and the data analysis unit 180, and the data analysis unit 180 is connected to the computer 110.

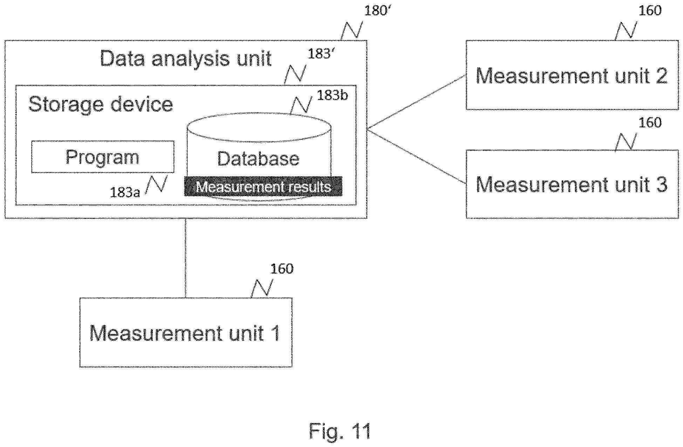

An embodiment may also be realized by a configuration in which the data analysis unit of one of the analyzers is utilized as the computer 110. That is, as shown in **Fig. 11,** the data analysis unit 180' of one of the analyzers 150 may be utilized as the computer 110 and be connected to each of the measurement units 160, and may have a function of receiving measurement data from each of all measurement units, processing the received measurement data using computer instructions in program (product) 183a to generate the measurement results for each of the measurement units and storing the measurement results of samples measured by each of the measurement units in a corresponding database 183b in the storage device 183'.

In addition, instead of a configuration in which measurement results of samples which had been measured by each of the measurement units 160 are collected by the computer 110 and are centrally managed by the database 116 in the computer 110, the measurement results may also be stored in multiple databases via distributed management. For example, the measurement results of samples which had been measured by the measurement unit M1 may be stored in a database in the storage device 183 of the data analysis unit 180 corresponding to the measurement unit M1, and the measurement results of samples which had been measured by the measurement unit M2 may be stored in a database in the storage device 183 of the data analysis unit 180 corresponding to the measurement unit M2, and when the inter-instrument difference evaluation is conducted, the computer 110 may access each of the databases and collect the measurement results of the target sample from each of the databases. According to this alternative embodiment, even if the databases are distributed across different devices, the computer 110 can search the databases to automatically collect the measurement results of the target sample and generate the evaluation information, thus being able to reduce operator's workload when conducting inter-instrument difference evaluation.

**Embodiment 2**

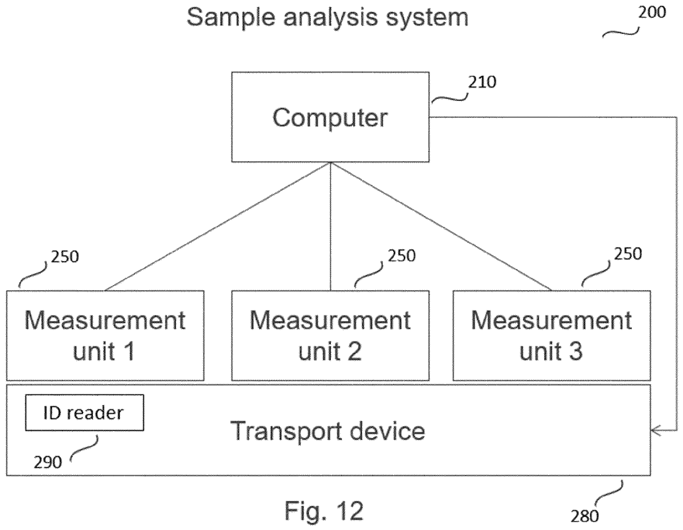

**Fig. 12** shows another sample analysis system 200 including a computer 210, a plurality of measurement units 250 corresponding to respective analyzers, and a transport device 280. The configuration of the computer 210 corresponds to the configuration of the computer 110 described above in conjunction with **Fig. 2.** In addition, the configuration of the measurement unit 250 corresponds to the configuration of the measurement unit 160 described above in conjunction with **Figs. 3** and **4.** The transport device 280 may be configured to transport the sample container or a sample rack holding at least one sample container to at least one of the measurement units 250. The sample container may contain the target sample (second sample) or the first sample. The transport device 280 may include an ID reader 290 (identification information reader), such as the barcode reader, for reading the ID attached to the sample container. The ID reader 290 may be located at the transport start position of the transport device 280 to read the IDs from the sample containers at the transport start position. Operations of the transport device 280 may be controlled by the computer 210, which may be communicatively connected to the ID reader 290. The ID reader 290 may read ID of each of a plurality of first samples to be measured by at least one of the measurement units 250. This ID information may be then transmitted to the computer 210, and the computer 210 may designate the second sample (target sample) from the plurality of first samples whose ID has been read by the ID reader 290. The transport device 280 may transport the sample container of the target sample (second sample) to each of the plurality of target measurement units 250 which are subject for the inter-instrument difference evaluation. For example, the computer 210 may autonomously designate the target sample from the plurality of first samples, and may control the transport device 280 so that the designated target sample is transported to the target measurement units 250. The computer 210 may not be just a single computer unit, but also be distributed computers or distributed computer units that may include a transport controller that controls the transport device 280 and a measurement unit controller that controls the respective measurement units 250.

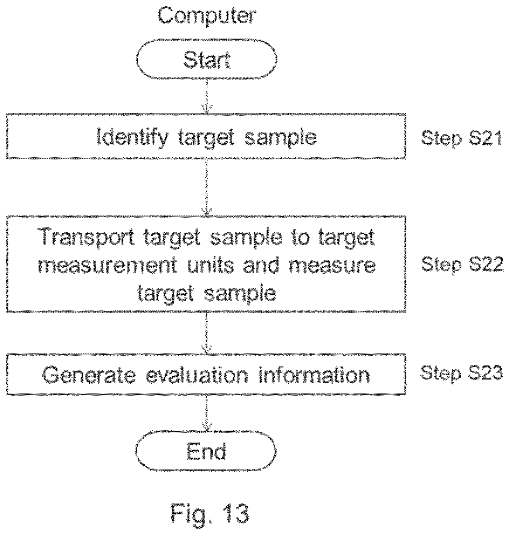

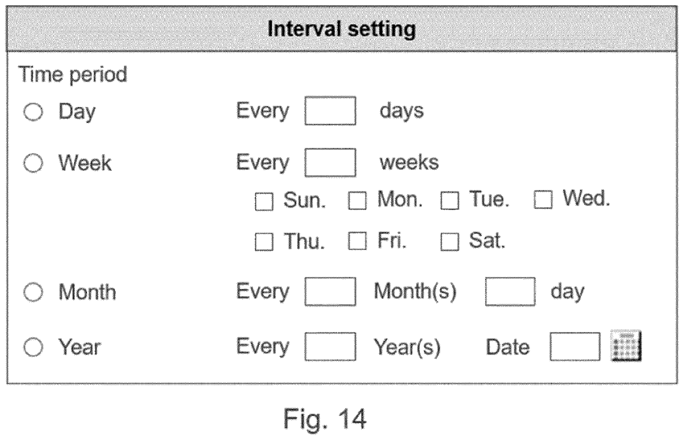

**Fig. 13** shows an exemplary process according to this sample analysis system 200. According to step **S21** of **Fig. 13,** the computer 210 may autonomously identify (designate), by using one or more set conditions, the target sample from a plurality of the first samples whose ID has been read by the ID reader 290. For example, the computer 210 may automatically designate the target sample based on a predetermined timing for conducting the measurements for the inter-instrument difference evaluation. As indicated in **Fig. 14** showing a plurality of settable conditions on a schedule of the measurement for the inter-instrument difference evaluation, the predetermined timing may be preset and modified via the graphical user interface as exemplary shown in the **Fig. 14.** For example, the computer 210 may receive the setting of intervals such as time periods. As the time period, the computer 210 may receive settings such as once a year, one a month, every day, etc. If the setting of the interval is every day, for example, the computer 210 may designate the first sample in each day as the target sample. For example, when the timing has arrived, the computer 210 may randomly designate the target sample from the first samples whose ID has been read by the ID reader 290. Then, in step **S22** of **Fig. 13,** the computer 210 may control the transport device 280 to transport the designated target sample to each of the target measurement units 250 for the measurements for the inter-instrument difference evaluation. At each of the target measurement units 250, a portion of the target sample is aspirated and measured. The measurement results of the target sample obtained by the target measurement units 250 are stored in the computer 210. Then, in step **S23** of **Fig. 13,** the computer 210 may generate evaluation information based on the set of measurement results of the target sample measured by each of the target measurement units 250. Note that, for samples used for a routine measurement for diagnosis of test subjects, the computer 210 may determine a destination measurement unit according to a measurement order of the sample for diagnosis of the test subject and may control the transport device 280 to transport a sample container including the sample to the destination measurement unit 250.

In step **S21,** if a condition on a quantity of the target sample has also been set in the sample analysis system 200, for example, the computer 210 may also determine whether the sample container contains sufficient sample volume for the measurements by each of the target measurement units 250. The sample volume may be detected, for example, using an image sensor which captures a side view of the sample container. The captured image may be transmitted to the controller 161, which determines the liquid level through edge and contour detection of the image, then calculates the volume based on the container's geometry. If the sample volume does not meet the condition, the computer 210 may identify a different sample as the target sample. This initial determination of a sufficient sample volume advantageously prevents termination of measurements for the purpose of inter-instrument difference evaluation due to insufficient sample volume.

In step **S21,** the computer 210 may designate, as the target sample, the sample whose measurement order includes measurement items which are suitable for the inter-instrument difference evaluation. For example, among a plurality of first samples whose ID has been read by the ID reader 290, if there is the sample whose measurement order for the diagnosis of the test subject includes measurement items which also cover measurement items for the inter-instrument difference evaluation, the computer 210 may designate this sample as the target sample since the measurement result of the sample can also be utilized to the inter-instrument difference evaluation. If the measurement item of the measurement order can only be measured by a specific measurement unit, the computer 210 may exclude such sample from being the target sample since such a sample is not suitable for the inter-instrument difference evaluation. For example, the system 200 may include the specific measurement unit suitable for a specific measurement item, and there may be only one or a few of the specific measurement units. If a sample's measurement order includes only the specific measurement item, the sample is measurable only by the specific measurement unit. Thus, if such sample is designated as the target sample, the target measurement units may not be able to measure the sample. Therefore, such sample is not suitable for the inter-instrument difference evaluation.

According to the process of **Fig. 13,** the computer 210 autonomously designates the target sample, and the computer 210 controls the transport device 280 so that the designated target sample is transported to each of the target measurement units 250 for inter-instrument difference evaluation. Therefore, automation can be further increased, and it is possible to further reduce operator's workload when performing measurement for inter-instrument difference evaluation.

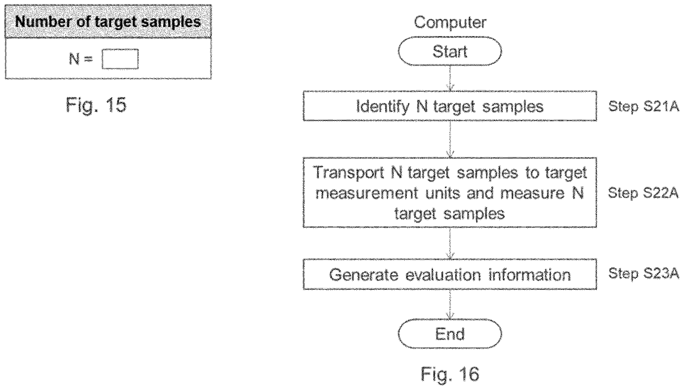

The computer 210 may additionally receive a setting of number of target samples. The number may be an integer greater than or equal to 2. If only a single target sample is measured by each of the target measurement units 250, measurement values of the single sample may fluctuate depending on which part of the sample within the sample container is aspirated for measurement, especially when the concentration of blood cells is unevenly distributed in the sample. In order to reduce the impact of the fluctuations on measurement result to be used for the inter-instrument difference evaluation, the computer 210 may be configured to receive a setting of the number of target samples N to, for example, obtain an average value of the total measurement values for the N target samples. The operator may enter the number of target samples N such as 20 for statistical calculation through a setting screen of a graphical user interface, as shown in **Fig. 15**. The skilled person understands that this improves the accuracy of the inter-instrument difference evaluation. The number of target samples N may be fixed at a default value.

**Fig. 16** shows a process to generate evaluation information when the computer 210 has received the setting of number of target samples N. In step **S21A** of **Fig. 16,** for example, the computer 210 may identify N target samples from a plurality of first samples whose ID has been read by the ID reader 290. Then, in step **S22A** of **Fig. 16,** the computer 210 may control the transport device 280 to transport each of the identified N target samples to each of the target measurement units 250 for measurement for the inter-instrument difference evaluation. At each of the target measurement units 250, each of N target samples is aspirated by the target measurement unit and measurement of each target sample is conducted. The measurement results of each of the N target samples are stored in the computer 210. Then, in step **S23A** of **Fig. 16,** the computer 210 may generate evaluation information by statistically calculating measurement values of the N target samples.

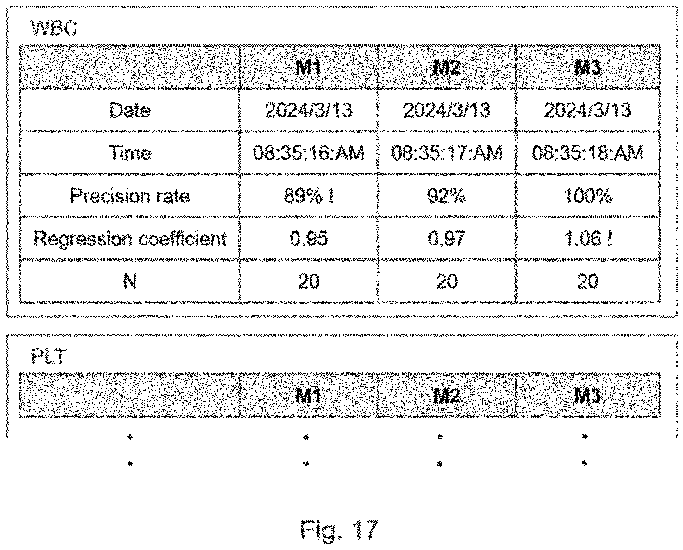

For example, evaluation information may be output as shown in **Fig. 17** for each measurement item. While this example is described for the measurement item WBC when N (number of target samples) is 20, the skilled person understands that the evaluation information may equally be provided for other measurement items and/or for different number of target samples N. In this example, a precision rate and a regression coefficient may be statistically calculated for each of the target measurement units M1 - M3. When the calculated value is outside an allowable range, an indication such as an error mark may be displayed. For example, since the precision rate of the measurement unit M1 is 89% which is below a threshold value 90%, the exclamation mark ("!") is added at the display area of the precision rate 89%. The precision rate and the regression coefficient may be calculated by the computer 210 as follows:

Precision rate:

(1) For each target sample, the computer 210 may calculate the match rate indicating how well the measured value by each target measurement unit matches the standard value. The match rate m may be calculated based on the equation below:

$$m = \left| \frac{(y - x)}{x} \right| \times 100$$

where y is the measured value by each target measurement unit and x is the standard value. x may be the measured value by the master device, or x may be an average of measured values obtained by each of the target measurement units.

(2) For each of the target measurement units, the computer 210 may count the number of target samples whose match rate is within an allowable range, and the counted number is represented as G.

(3) For each of the target measurement units, the computer 210 may calculate a ratio between the G and N and obtain the precision rate P based on the equation below:

$$P = \frac{G}{N}$$

If the precision rate P is greater than or equal to a threshold value such as 90%, the target measurement unit is determined to pass the inter-instrument difference evaluation.

Regression coefficient:

When the master device has been set in the sample analysis system 200, the computer 210 may calculate master device average, measurement unit average, standard deviation, and covariance using the following formula.

$$\bar{x} = \frac{1}{N} \sum_{i=1}^{N} x_i \, ,$$

$$\bar{y} = \frac{1}{N} \sum_{i=1}^{N} y_i \, ,$$

$$SD_x = \sqrt{\frac{1}{n} \sum_{i=1}^{n} (x_i - \bar{x})^2}$$

$$SD_y = \sqrt{\frac{1}{n} \sum_{i=1}^{n} (y_i - \bar{y})^2}$$

$$C_{xy} = \frac{1}{n} \sum_{i=1}^{n} (x_i - \bar{x})(y_i - \bar{y})$$

where $x_1,...,x_N$ are the measured values of the master device; $y_1,...,y_N$ are the measured values of the target measurement unit other than the master device; $SD_x$ is the standard deviation of the master device; $SD_y$ is the standard deviation of the target measurement unit other than the master device; $C_{xy}$ is the covariance.

The computer 210 may calculate a correlation coefficient using the following formula based on the covariance and standard deviation.

$$r = \frac{C_{xy}}{SD_x SD_y}$$

The computer 210 may calculate the regression coefficient using the following formula based on the correlation coefficient and standard deviation.

$$a = r \times \left( SD_y \div SD_x \right)$$

where a is the regression coefficient and r is the correlation coefficient.

When the master device has not been set in the sample analysis system 200, the computer 210 may calculate an average of the measurement values from each of the target measurement units and set the aver-

age values as $x_1,...,x_N$.

In the configuration where the computer 210 identifies the N target samples to generate the evaluation information, the computer 210 may selectively identify the N target samples which possess a specific attribute. This attribute may refer to a specific characteristic or property of the sample. The computer 210 may be configured to distinguish the attribute of each target sample based on a measurement result of the target sample which has been already measured by at least one of the measurement units 250 for diagnosis of a test subject. That is, this configuration is based on the sample analysis system 200 where the target sample is selected from a plurality of first samples after they have been measured by at least one of the measurement units 250. One such attribute may be a cell concentration indicating a blood cell concentration of the target sample. For example, the computer 210 may selectively identify the N target samples with low cell concentration (below a first threshold). The cell concentration used as the attribute may not only be low but also middle (between the first threshold and a second threshold) or high (above the second threshold). The specific attribute for selectively identifying the N target samples may be information indicating whether a normal or abnormal measurement value has been obtained, or whether an abnormal feature has been detected as a result of the measurement.

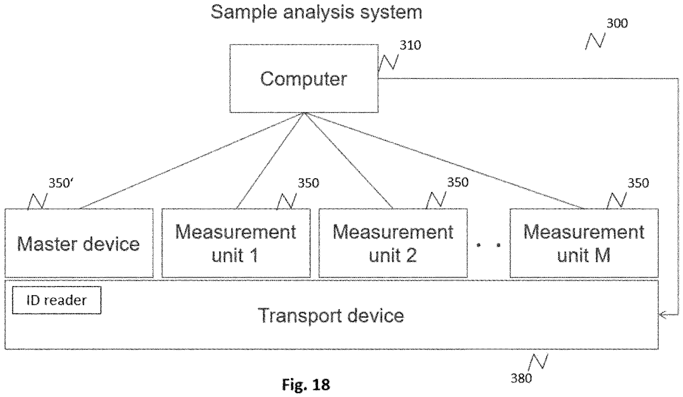

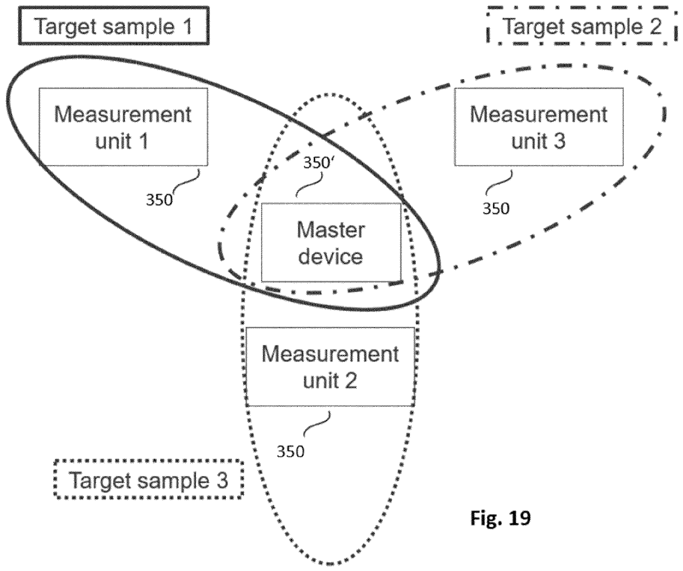

In a facility with a plurality of measurement units such as dozens of measurement units, it may be impractical to conduct the inter-instrument difference evaluation measurement using a single sample container for all the target measurement units in the facility. To address this issue, the computer may be configured to control a sample analysis system so that the deviation of each target measurement unit relative to a master device can be calculated, allowing the inter-instrument difference among all the target measurement units to be evaluated based on these calculated deviations. **Fig. 18** illustrates an example of a sample analysis system 300 including a master device (which is a master measurement unit) 350' and additional measurement units 1, 2, ..., M, all indicated with reference sign 350, which may be serviced by a transport device 380 which may be controlled by the computer 310 as described above. In this embodiment, the computer 310 may generate a plurality of groups, each including the master device 350' and one or more target measurement units 350. The number of the measurement units in each group is preferably limited to a number that can be covered by the single sample container. **Fig. 19** shows an example of the groups, each including the master device 350' and another measurement unit 350 as a pair. The computer 310 may allocate each of different target samples to each of the groups so that the measurement units in the same group can measure the same target sample. Then, the computer 310 may control the transport device 380 so that each target sample is transported to each of the measurement units in each group and measured for evaluation of inter-instrument difference in each group. According to the example shown in **Fig. 19,** a first target sample 1 may be allocated and measured for evaluation of inter-instrument difference between the master device 350' and measurement unit 1, a second target sample 2 may be allocated and measured for evaluation of inter-instrument difference between the master device 350' and measurement unit 3, a third target sample 3 may be allocated and measured for evaluation of inter-instrument difference between the master device 350' and measurement unit 2. The computer 310 may then calculate the difference of measurement results between the master device 350' and the corresponding one or more measurement units 350 in each group to evaluate whether the measurement units in each group can demonstrate equivalent measurement performance. If the difference of measurement results between the master device 350' and the corresponding one or more measurement units 350 in each group is within an allowable range, the computer 310 may determine that all of the target measurement units can produce equivalent measurement results.

Instead of generating the group including the master device 350' and other measurement unit 350 as a pair as illustrated in **Fig. 19,** the computer 310 may generate a plurality of groups, each including the master device 350' and other two or more measurement units 350.

According to the alternative embodiments shown by **Fig. 18** and **Fig. 19,** even if the number of the target measurement units 350 makes it impractical to perform the measurement for inter-instrument difference evaluation using a single sample container for all the target measurement units 350, it is possible to perform measurements for evaluating the inter-instrument difference among all the target measurement units 350 without requiring an operator to perform tasks such as picking up a plurality of target samples, dividing the target measurement units 350 into groups, allocating each target sample to a group and manually transporting each target sample to respective groups.

**Embodiment 3**

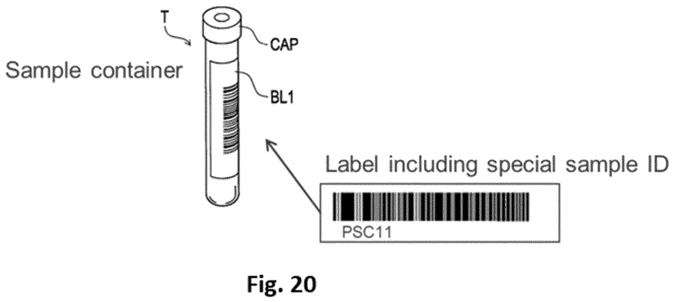

In this embodiment of the present disclosure, a label including a unique identifier for uniquely indicating a sample for the measurement for the inter-instrument difference evaluation may be attached onto a sample container containing the target sample so that the sample analysis system can automatically identify the target sample. For example, as shown in **Fig. 20,** a label BL1 may be attached onto the sample container T containing the target sample. The label BL1 may include, as the unique identifier, a special sample ID specifically for the measurement for the inter-instrument difference evaluation. The special sample ID may be created by combining predetermined set of letters, such as "PSC", with a sample ID number of the target sample. The unique identifier does not have to be special. For example, a specific set of last three digits of the ID (e.g., "717") may be preset as the unique identifier in the computer. If the ID of the sample read by the ID reader of the sample analysis system includes the last three digits corresponding to the preset values, the sample may be recognized as the target sample. The preset values may be determined according to the facility's ID assignment rules and may include a combination of letters and numbers.

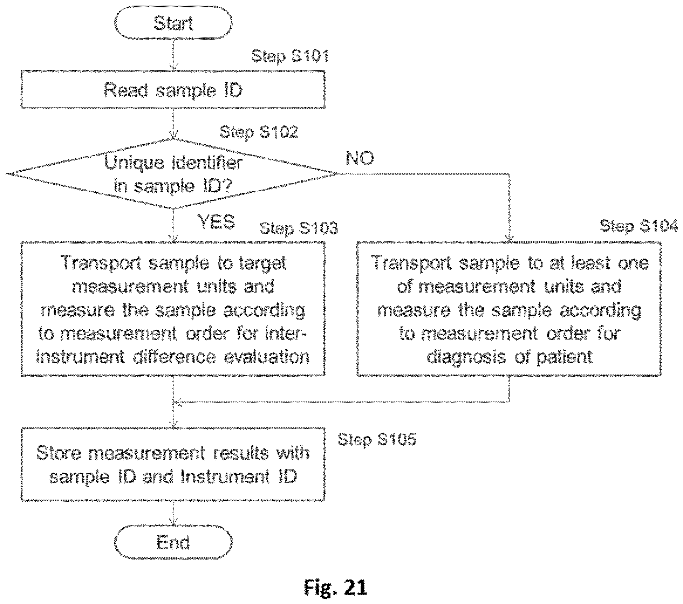

**Fig. 21** shows an exemplary process of this embodiment based on the sample analysis system 200 shown in **Fig. 12.** In step **S101,** the ID may be read by the ID reader 290 from a label of a sample container at the transport start position of the transport device 280. If the computer 210 has determined that the ID read by the ID reader 290 includes the unique identifier in step **S102,** the computer 210 may determine that the timing to conduct measurement for the inter-instrument difference evaluation has arrived, and the computer 210 may identify the sample in the sample container as the target sample and, in step **S103,** may control the transport device 280 to transport the sample container to each of the target measurement units 250. The computer 210 may read out the measurement order including the preset one or more measurement items for the inter-instrument difference evaluation and transmit it to each of the target measurement units 250. At each of the target measurement units 250, a portion of the same target sample may be aspirated, and preparation of measurement samples and measurement for the same measurement items are conducted. On the other hand, if the computer 210 has determined that the sample ID read by the ID reader 290 does not include the unique identifier in step **S102,** the computer 210 may control the transport device 280 to transport the sample container to at least one of the measurement units 250 in step **S104.** In step **S104,** the computer 210 may read out this sample's measurement order that has been registered for diagnosis of a patient. Then, the computer 210 may determine a destination measurement unit 250 based on the measurement order of the sample, availability information of each measurement unit 250, etc., and may control the transport device 280 to transport the sample container to the destination measurement unit 250. The computer 210 may transmit the measurement order to the destination measurement unit 250 so that the measurement of the sample is conducted according to the measurement order. Then, in step **S105,** the measurement result of the measured sample is stored in database 116 of the computer 210 in association with the ID and Instrument ID. Here, the measurement results of the target sample are stored in the database 116 in association with the sample ID including the unique identifier.

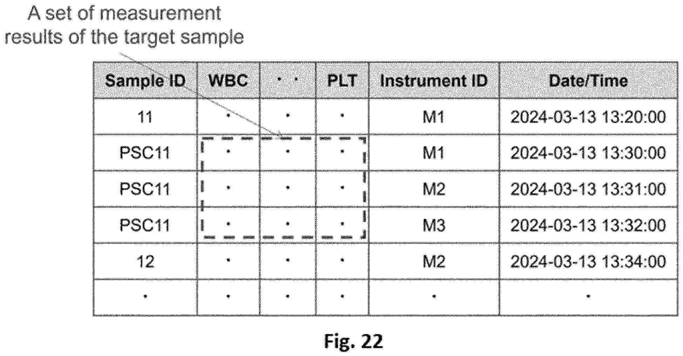

**Fig. 22** shows an exemplary data structure of the database 116 in which measurement results are stored in association with the ID and Instrument ID. The measurement results obtained by routine measurements for diagnosis of patients are stored in association with the ID which is a regular format such as "12". The measurement results obtained by measurement for the inter-instrument difference evaluation are stored in association with the ID including the unique identifier such as "PSC11". When generating the evaluation information, the computer 210 may search for measurement results of the target sample corresponding to the unique identifier "PSC11" and Instrument IDs "M1", "M2" and "M3". This unique identifier can be used as search information for searching for the measurement results of the target sample.

According to this embodiment, the computer 210 identifies the target sample based on the ID read by the ID reader 290 at the transport device 280, and controls the transport device 280 to transport the identified target sample to each of the target measurement units 250. Therefore, it is possible to automatically perform measurement for the inter-instrument difference evaluation simply by placing the sample container with unique identification information onto the transport device. This reduces the need for operator intervention.

The sample analysis system 200 may also be utilized in a case where the ID of the target sample is designated by the operator and the designated ID is stored in the storage device of the computer 210. In this embodiment, in step **S101,** the computer 210 may receive the ID read by the ID reader 290 from a sample container, and in step **S102,** may determine whether the ID read by the ID reader 290 matches the ID designated by the operator as the target sample. When the IDs match, the computer 210 may identify the target sample, and in step **S103,** may transport the sample container to each of the target measurement units 250 for measurement for inter-instrument difference evaluation.

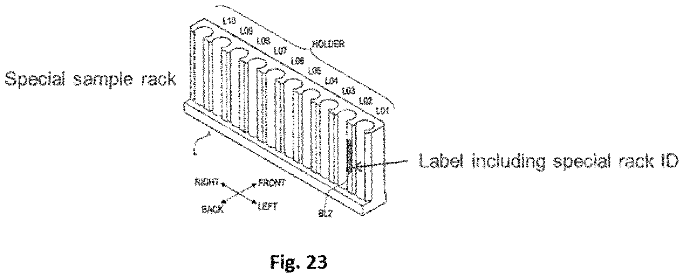

A label including the unique identifier may be attached onto a sample rack which can hold one or more sample containers. **Fig. 23** shows an example of the sample rack having a label BL2 including, as the unique identifier, a special rack ID specifically for the measurement for the inter-instrument difference evaluation. The special rack ID may include a predetermined set of letters such as "PSCR". Alternatively, the unique identifier does not have to be special. For example, a specific set of last two digits of a rack ID (e.g., "55") may be preset as the unique identifier in the computer.

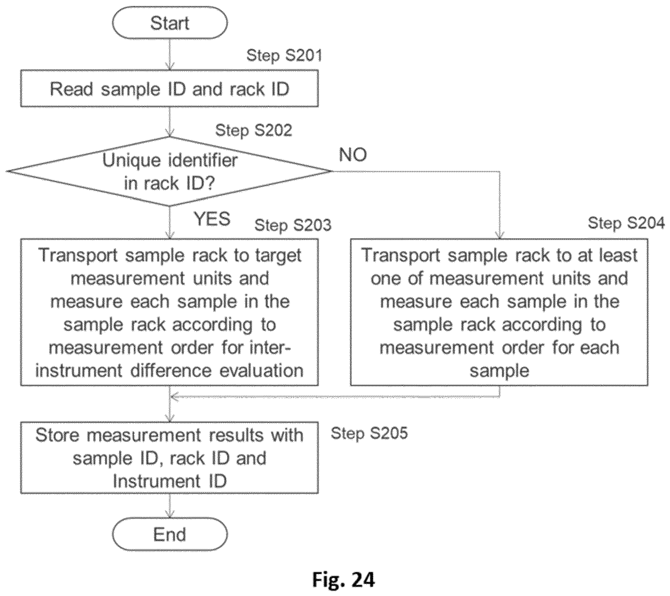

**Fig. 24** shows an exemplary process based on the sample analysis system 200 shown in **Fig. 12.** In step **S201,** at the transport start position of the transport device 280, the rack ID may be read from a label of a sample rack by the ID reader 290 and the ID of the sample may be read from a label of each sample container in the sample rack by the ID reader 290. If the computer 210 has determined that the rack ID read by the ID reader 290 includes the unique identifier in step **S202,** the computer 210 may determine that the timing to conduct measurement for the inter-instrument difference evaluation has arrived, and the computer 210 may identify each sample in the sample rack as the target sample and, in step **S203,** the computer 210 may control the transport device 280 to transport the sample rack to each of the target measurement units 250. The computer 210 may read out the measurement order including a preset one or more measurement items for the inter-instrument difference evaluation and transmit it to each of the target measurement units 250. At each of the target measurement units 250, a portion of the target sample contained in each sample container in the sample rack may be aspirated, and preparation of measurement samples and measurement for the same measurement items are conducted according to the measurement order. On the other hand, if the computer 210 has determined that the rack ID read by the ID reader 290 does not include the unique identifier in step **S202,** the computer 210 may control the transport device 280 to transport the sample rack to at least one of the measurement units 250 in step **S204.** In step **S204,** the computer 210 may, for each sample in the sample rack, read out a measurement order that has been registered for diagnosis of a patient. Then, the computer 210 may determine a destination measurement unit 250 based on the measurement order of each sample, availability information of each measurement unit 250, etc., and may control the transport device 280 to transport the sample rack to the destination measurement unit 250. The computer 210 may transmit the measurement order for each sample to the measurement unit 250 so that each sample in the sample rack is measured according to the measurement order. Then, in step **S205,** the measurement result of the measured sample is stored in database 116 of the computer 210 in association with the ID of the sample, the rack ID and the Instrument ID. The measurement results of the target sample are stored in the database 116 in association with the rack ID including the unique identifier.

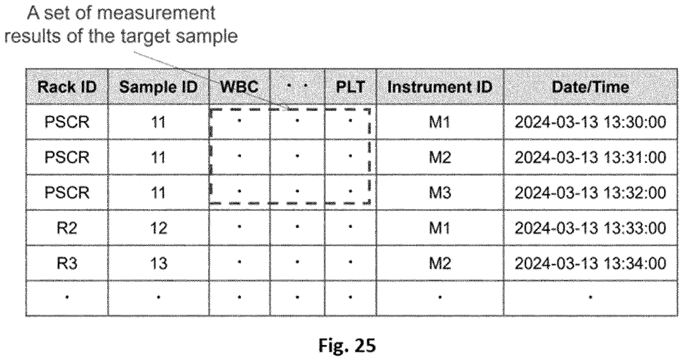

**Fig. 25** shows an exemplary data structure of the database 116 in which measurement results are stored in association with the ID of the sample, the rack ID and the Instrument ID. The measurement results obtained by routine measurements for diagnosis of patients are stored in association with the ID and the rack ID which are defined with the regular form such as "R2" or "R3". The measurement results obtained by measurement for the inter-instrument difference evaluation are stored in association with the rack ID including the unique identifier such as "PSCR". To generate the evaluation information, the computer 210 may search for a set of measurement results of the target sample corresponding to the unique identifier "PSCR" and the Instrument IDs "M1", "M2" and "M3".

According to this embodiment, the computer 210 identifies one or more target samples based on rack identification information read by the ID reader 290 at the transport device 280, and controls the transport device 280 to transport the sample rack holding the identified one or more target samples to each of the target measurement units 250. Therefore, it is possible to automatically perform measurement of one or more samples for the inter-instrument difference evaluation simply by placing the sample rack with unique identification information onto the transport device. This reduces the need for operator intervention.

The sample analysis system 200 may also be utilized in a case where the rack ID of the sample rack for holding one or more target samples is designated and the designated rack ID is stored in the storage device of the computer 210. In this embodiment, in step **S201,** the computer 210 may receive the rack ID read by the ID reader 290 from a sample rack, and in step **S202,** the computer 210 may determine whether the rack ID read by the ID reader 290 matches the designated rack ID. When the rack IDs match, the computer 210 may identify the one or more target samples in the sample rack, and in step **S203,** may transport the sample rack to each of the target measurement units 250 for measurement for inter-instrument difference evaluation.

Other unique information instead of the above-explained unique identifier may also be used to distinguish the sample rack corresponding to the inter-instrument difference evaluation measurement. The unique information may be color information of a unique color which indicates that the rack is used for a measurement for the inter-instrument difference evaluation. The unique color may be different from a color of a normal rack for routine measurements for diagnosis of patients. The color information of the unique color may be pre-set in the computer. In this alternative approach, when conducting the measurement for the inter-instrument difference evaluation, an operator may place one or more sample containers, each containing the target sample, into the sample rack with the unique color and may place this sample rack at the transport start position of the transport device. In order to detect the color of the sample rack, the transport device may include, at the transport start position, an image sensor which is configured to capture an image of a predefined region of the sample rack. The captured image may be transmitted to the controller 161, which analyzes the image and extracts the color components of the image pixels. The controller 161 may obtain average values of the red (R), green (G) and blue (B) color values of the pixels and identify the color of the sample rack by comparing these average values with reference data values. The transport device may include a color sensor configured to detect the color of the sample rack. The color sensor emits light from a light emitter to the sample rack and then receives light reflected back from the sample rack with a light receiver. The color sensor may measure the intensity of the reflected light for each of red, green and blue wavelengths, calculate a ratio of the intensities of the red, blue and green light, and identify the color of the sample rack by comparing the calculated ratio with reference ratios. The detection result of the color of the sample rack may be transmitted to the computer, which then determines whether the color of the sample rack corresponds to the unique color. When the computer has determined that the color of the sample rack matches the unique color, the computer may identify each sample in the sample rack as the target sample and control the transport device to transport the sample rack to each of the target measurement units. When measurement results of the target sample are obtained by each of the target measurement units, the measurement results are stored in database of the computer in association with a flag indicating the unique color, the ID of the sample and the instrument ID so that the computer can search for measurement results corresponding to the flag from the database.

Alternatively, instead of using the transport device, the sample container containing the target sample may be manually transported to each of the target measurement units by the operator. The operator may pick up a sample container which contains the sample suitable for conducting measurement for the inter-instrument difference evaluation by observing a color or volume of the sample. The operator may attach the label including the unique identifier, such as the label BL1, onto the sample container and then manually transport the sample container to each of target measurement units that are subject to the inter-instrument difference evaluation.

At each of target measurement units, the ID including the unique identifier may be read from the label of the sample container by the ID reader 165 and the sample ID may be transmitted to the computer. The computer may identify the target sample based on the unique identifier and transmit the measurement order for the measurement for the inter-instrument difference evaluation to each of the target measurement units so that the target sample is measured by each of the target measurement units for one or more same/common measurement items. The measurement results obtained based on measurements by each of the target measurement units may be stored in database 116 of the computer in association with the ID including the unique identifier.

The sample rack holding one or more target samples may be manually transported to each of the target measurement units by the operator. The operator may pick up one or more sample containers, each containing the sample suitable for conducting measurement for the inter-instrument difference evaluation, place the picked-up one or more sample containers in the sample rack having the label including the unique identifier, such as the rack label BL2, and then manually transport the sample rack to each of the target measurement units.

At each of the target measurement units, the rack ID including the unique identifier may be read from the label of the sample rack by the ID reader 165 and a sample ID may be read from each sample container in the sample rack by the ID reader 165. These IDs may be transmitted to the computer. The computer may identify each sample in the sample rack as the target sample based on the unique identifier included in in the rack ID and transmit the measurement order for the measurement for inter-instrument difference evaluation to each of the target measurement units so that each target sample in the sample rack is measured by each of the target measurement units for one or more same/common measurement items. The measurement results obtained based on measurements by each of the target measurement units may be stored in database 116 of the computer in association with the rack ID including the unique identifier.

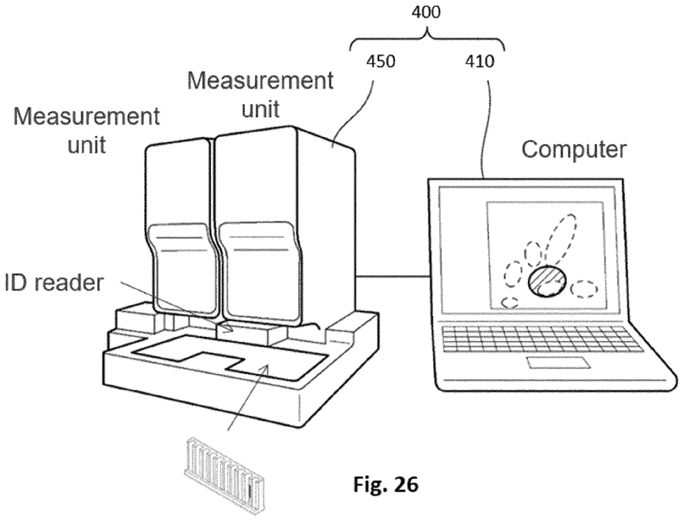

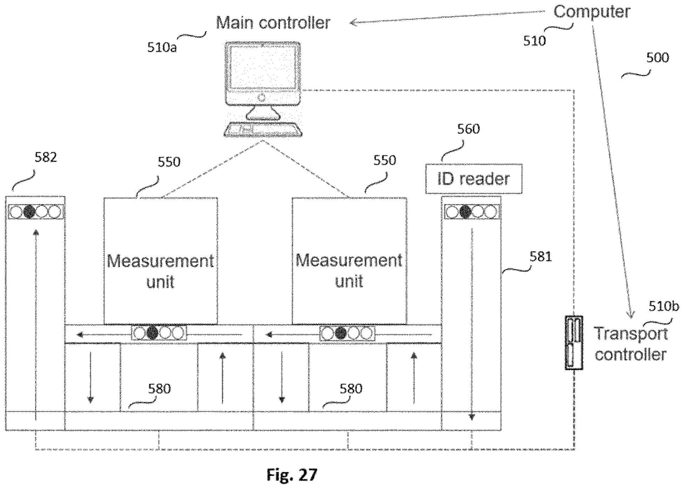

The present disclosure related to the transport device may further be exemplified by the configuration of a sample analysis system 400 as shown in **Fig. 26** or the configuration of a sample analysis system 500 as shown in **Fig. 27.**

In the configuration as shown in **Fig. 26,** the sample analysis system 400 may include multiple measurement units 450 being adjacently arranged along a single transport device. The transport device may include: a rack placement section where a sample rack is placed by the operator; a transport line that receives the sample rack from the rack placement section and transports the sample rack to at least one of the multiple measurement units 450 that are arranged along the transport line; and a rack receiving section that receives the sample rack from the transport line. The transport device may be equipped with an ID reader which reads ID before the sample container is transported to any of the measurement units 450. The computer 400 may control operations of the transport device and the measurement units based on the ID read by the ID reader.

In the configuration of the sample analysis system 500 as shown in **Fig. 27,** multiple measurement units 550 and multiple transport devices 580 may be arranged in one-to-one correspondence. The multiple transport devices 580 are connected to each other. A rack loading device 581 may be arranged in an upstream side of the transport devices 580. Sample racks may be placed by the operator onto the rack loading device 581 and may be loaded by the rack loading device onto the transport device 580 on the upstream side. ID reader 560 may be installed in the rack loading device so that the ID can be read before the sample racks are loaded from the rack loading device 581 into the transport device 580. A rack collecting device 582 may be arranged in a downstream side of the transport devices 580. Operations of the multiple transport devices 580, the rack loading device 581 and the rack collecting device 582 may be controlled by the computer 510 (a main controller 510a and a transport controller 51 0b) based on the ID read by the ID reader 560. The transport operations may be controlled by the transport controller 510b and the measurement operations may be controlled by the main controller 510a. As further indicated in **Fig. 27,** a sample rack may include a plurality of samples including at least one target sample, and the sample analysis system 500 may be controlled to transport the at least one target sample via the multiple transport devices 580 to each of the multiple measurement units 550 for the purpose of evaluating the inter-instrument difference.

**Embodiment 4**

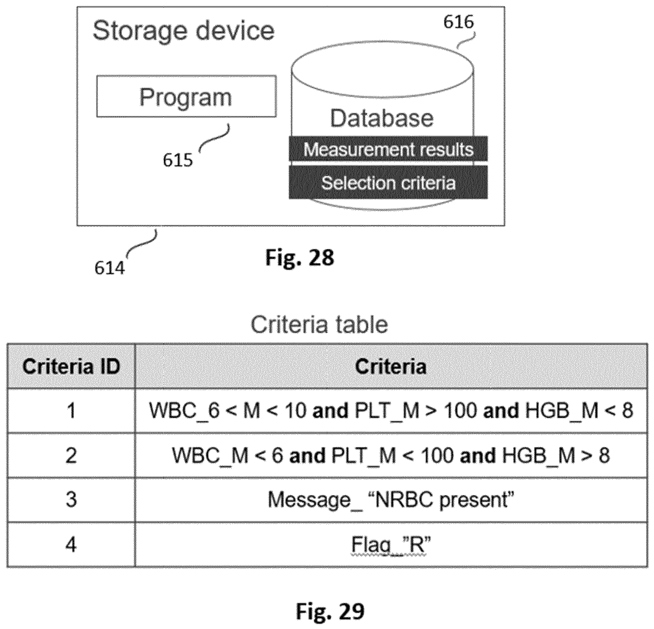

In this embodiment of the present disclosure, the computer 110, 210, 310, 410, 510 may automatically designate the target sample suitable for the measurement for the inter-instrument difference evaluation, based on or using measurement results of a plurality of samples which have already been measured by at least one of the measurement units. That is, the computer 110, 210, 310, 410, 510 may designate the target sample from samples which have been measured by at least one of the measurement units. In other words, the computer may designate, as the second sample (target sample), one of a plurality of the first samples that have already been measured by at least one of the plurality of the measurement units. For example, the computer may designate the target sample according to the measurement result of the first sample that has already been measured by at least one of the plurality of measurement units 150, 250, 350, 450, 550. In particular, the the target sample may be designated by selecting the first sample whose measurement result meets one or more selection criteria. As shown in **Fig. 28,** the one or more selection criteria may be stored in the database 616 of the storage device 614 of the computer. The storage device 614 of the computer may also store a program (product) 615. The one or more selection criteria may be stored as an exemplary form of criteria table, for example in the form of a data structure or the like.

**Fig. 29** shows an exemplary criteria table indicating examples of the one or more selection criteria. As shown in **Fig. 29,** the criteria table may store defined criteria, including a range of values. The range of values may indicate a normal value range so that normal samples can be selected as the target samples. Conversely, the range of values may indicate an abnormal value range so that abnormal samples can be selected as the target samples. This allows the operator to verify whether the target measurement units can equivalently measure the abnormal samples and produce measurement results within the abnormal value range. The criteria may also include one or more messages and/or one or more alert flags that may be produced as a result of the measurement. By selecting the abnormal samples that meet the criteria, the operator is able to verify whether the target measurement units can equivalently measure these abnormal samples and produce the same messages or alert flags. The criteria table may also include additional various criteria. In the exemplary criteria table of **Fig. 29,** the criteria of ID "1" is defined for identifying samples whose measurement results (here, with regard to WBC, PLT, HGB) are within normal value ranges, the criteria of ID "2" is defined for identifying samples whose measurement results are within abnormal value ranges, the criteria of ID "3" is defined for identifying samples whose measurement results have triggered the message representing specific measurement result such as "NRBC present", and the criteria of ID "4" is defined for identifying samples whose measurement results have triggered the alert flag. The message "NRBC present" may be produced if NRBC (Nucleated Red Blood cells) values or percent valued thereof exceed a threshold. The alert flag may be produced if the measurement results are suspicious. The criteria may also include messages or alert flags related to immature granulocytes, primitive cells, anemia, iron deficiency anemia, trilineage reduction, thrombocytopenia, thrombocytosis, etc. The skilled person understands that these are non-limiting examples of the one or more selection criteria.

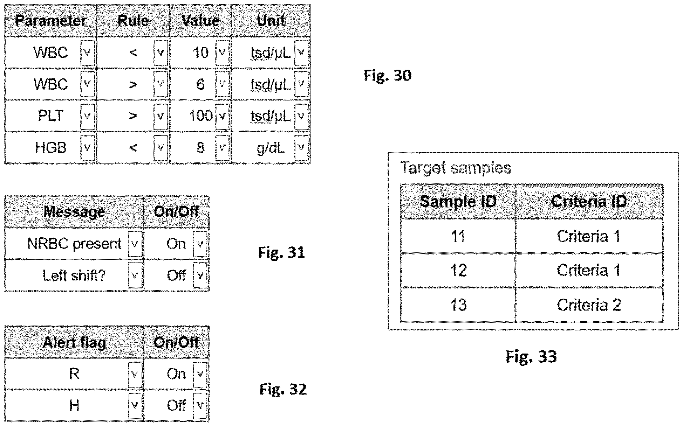

The selection criteria may be set by the operator of the sample analysis system 100, 200, 300, 400, 500. As shown in **Fig. 30** indicating an exemplary graphical user interface for setting the selection criteria, the operator may set a parameter by selecting, from a drop-down list, e.g. "WBC" in the field of parameter, may further define one or more logical operators by selecting, from a drop-down list, including a plurality of logical operators such as $\{>, \geq, <, \leq, =\}$, e.g. "<", and may enter (numerical) value, e.g. 10, in the field of value. The physical unit defined for the parameter may also be displayed automatically in the field of unit.

The operator may be able to add a new line, via the exemplary graphical user interface, to combine the multiple parameters. **Fig. 30** shows that four parameters may be combined to set a single criterium. As further shown in **Figs. 31** and **32,** the operator may also set ("ON") or unset ("OFF") messages and flags as additional criteria. For example, if the measurement result has the message which is set to "ON" as the criteria, the computer may select the sample corresponding to the result having such message.

For example, if the computer (110, 210, 310, 410, 510) has determined that a timing (e.g., the timing arriving in a predetermined frequency such as per day, per few days, per week, per few weeks, per month, per few months, etc.) to conduct the inter-instrument difference evaluation has arrived, the computer may autonomously/automatically select the target sample by applying the selection criteria to the measurement results stored in the database of the computer. For example, if the computer (110, 210, 310, 410, 510) has received an instruction from the operator to conduct the inter-instrument difference evaluation, the computer may initiate to compare the criteria and the measurement results measured after receiving the operator's instruction. If the computer finds that the measurement result matches the criteria, the computer may designate the first sample corresponding to the matched result as the target sample. **Fig. 33** shows a list of the selected target samples which may be displayed by the computer. The displayed information may include sample IDs of the selected target samples and/or the criteria IDs used for selecting the samples. The computer may store flag information indicating the inter-instrument difference evaluation in association with a sample ID of the selected sample in the database 616, as illustrated in **Fig. 8.**

In an exemplified case where the selected sample is placed on a transport device, a sample ID of the selected sample may be read by an ID reader located at or near the transport device and the computer may identify the selected sample as the target sample by comparing the sample ID read by the ID reader and the sample ID which is stored in association with the flag information. The computer may then control a transport of the identified target samples to each of the target measurement units by the transport device and send measurement instructions to each of the target measurement units to conduct the (same) measurement for inter-instrument difference evaluation.

In another exemplified case, the operator may refer to a display showing the list of **Fig. 33** and attach a label including the unique identifier to the sample container(s) containing the selected samples, or place the sample containers containing the selected samples into the sample rack onto which a label including the unique identifier is attached. When these samples or rack are placed on a transport device, the computer may identify the target sample based on the unique identifier which is read by ID reader located at the transport device.

According to this embodiment, the computer autonomously/automatically selects or searches for a sample suitable for the measurement for inter-instrument difference evaluation, based on the measurement result of the first sample and the selection criteria. This reduces the operator's workload for designating the target sample.

**Embodiment 5**

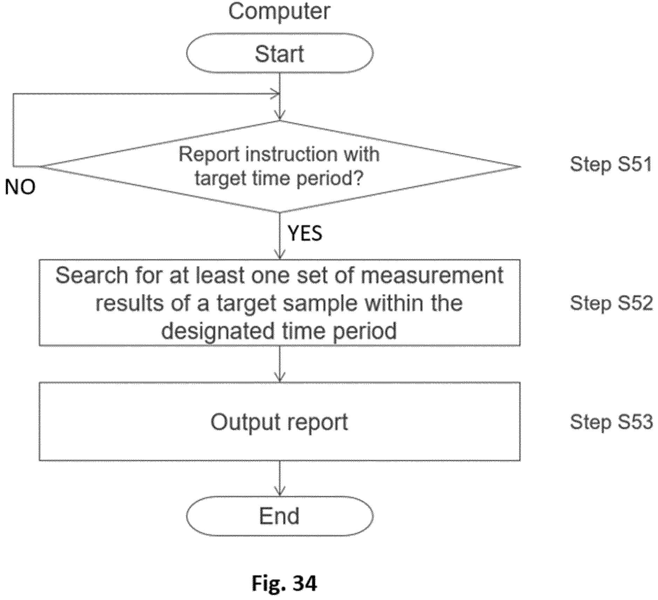

In this embodiment of the present disclosure, the computer 110, 210, 310, 410, 510 may generate a report as the evaluation information based on the measurement results stored in the database 116. This report may evaluate the equivalency of measurement results among multiple measurement units in a facility and may be used to document results of periodic evaluations of inter-instrument differences. **Fig. 34** shows a process to generate and output the report. In particular, as shown in the flowchart of **Fig. 34,** the computer, in step **S51,** may determine whether an operator's instruction to output a report has been received. The computer may be configured to receive the instruction together with a designation of a target time period. When the operator wants to refer to measurement results of one or more target samples which were measured on a date such as 2024/3/13, the operator can designate this date as the target time period. The computer may also be configured to receive the designation of time period of more than 1 day by receiving a starting date and an end date. Then, in step **S52,** the computer may search the database 116 for at least one set of measurement results of the one or more target samples within the designated time period. Then, in step **S53,** the computer may generate the report based on these retrieved measurement results and output the report. Instead of the time period, the computer may also be configured to receive the designation of the number of the target samples such as "20" to identify the designated number of the target samples going back from the current time.

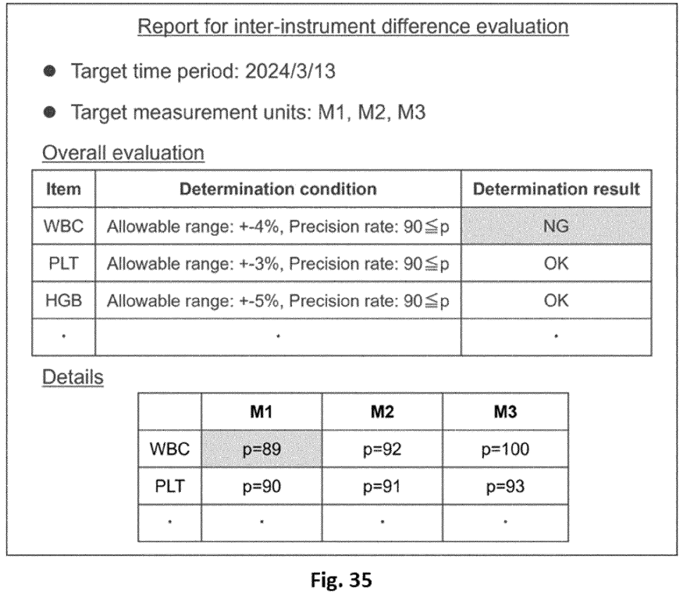

**Fig. 35** shows an example report for the inter-instrument difference evaluation. As shown in **Fig. 35,** the generated report may include the designated time period (here: 2024/3/31) and target measurement units (here: M1, M2, M3). The target measurement units are measurement units which were set as the target measurement units in the facility during the designated time period. Additionally, the report may also include the number of target samples that were measured during the designated time period. As shown in **Fig. 35,** the report may include sections for presenting overall evaluation results and detailed results. The overall evaluation section may include determination results and determination conditions for each measurement item of the preset one or more measurement items for the inter-instrument difference evaluation. The determination conditions are for evaluating whether measurement results from the respective target measurement units are equivalent to each other. The computer may determine a valid or acceptable inter-instrument difference ("OK") if measurement results of all target measurement units meet the determination conditions, and may determine an invalid or unacceptable inter-instrument difference ("NG") if measurement results of at least one of the target measurement units fail to meet the determination conditions. In the example shown in **Fig. 35,** the computer has determined "OK" for PLT measurements since measurement results of all target measurement units meet the determination conditions (Allowable range: +-3%, Precision rate: 90^p). However, for WBC measurements, the computer has determined "NG" since measurement results of at least one target measurement unit do not meet the determination conditions (Allowable range: +-4%, Precision rate: 90≦p). When the determination result is "NG", the operator can identify which measurement unit has caused this result by examining the details section of the report. In the example shown in **Fig. 35,** the operator can identify the measurement unit M1 as the cause of the "NG" result for WBC measurement item, as the WBC result from the measurement unit M1 is highlighted in the details section due to the precision rate of 89%, which does not meet the required condition of "Precision rate: 90≦p".

The operator may conduct a calibration of the measurement unit whose measurement result had violated the determination conditions and re-conduct measurement for the inter-instrument difference evaluation after the calibration of the measurement unit.

In the foregoing detailed description of the present disclosure, reference is made to the accompanying drawings that form a part hereof, and in which is shown by way of illustration how examples of the disclosure may be practiced. These examples are described in sufficient detail to enable those of ordinary skill in the art to practice the examples of this disclosure, and it is to be understood that other examples may be utilized and that process, electrical, and/or structural changes may be made without departing from the scope of the present disclosure.

Elements shown in the various figures herein can be added, exchanged, and/or eliminated so as to provide a number of additional examples of the present disclosure. In addition, the proportion and the relative scale of the elements provided in the figures are intended to illustrate the examples of the present disclosure and should not be taken in a limiting sense.

**Claims**

1. A sample analysis system (100) comprising:

   a plurality of measurement units (160), each configured to measure a sample to obtain a measurement result based on a measurement order including one or more measurement items;
   an identification information reader configured to read identification information of each of a plurality of first samples to be measured for diagnosis of a test subject by at least one of the plurality of measurement units; and
   a computer (110) that communicates with the identification information reader and the plurality of measurement units, wherein
   the first sample is measured by at least one of the plurality of measurement units based on a first measurement order for the diagnosis of the test subject, and a first measurement result of the first sample obtained from the at least one of the plurality of measurement units is stored, for use in the diagnosis of the test subject, in a storage device in association with the identification information of the first sample, and
   a plurality of target measurement units designated from the plurality of measurement units are subject for inter-instrument difference evaluation, wherein
   the computer is configured to:

   identify at least one of the plurality of first samples as a second sample used for the measurement for the inter-instrument difference evaluation, based on a timing to conduct the inter-instrument difference evaluation, wherein the timing is automatically determined by the computer; and
   control each of the plurality of target measurement units to measure the identified second sample based on a second measurement order including one or more preset measurement items for the inter-instrument difference evaluation, and store a plurality of second measurement results of the second sample obtained from the plurality of target measurement units, for use in the inter-instrument difference evaluation, in the storage device in association with the identification information of the second sample.

2. The sample analysis system (100) of claim 1, wherein each of the first samples is a sample collected from the test subject.

3. The sample analysis system (100) of claim 1 or 2, wherein the one or more preset measurement items comprise a plurality of measurement items which are common among the plurality of target measurement units.

4. The sample analysis system (100) of any one of claims 1 - 3, wherein

   the plurality of target measurement units comprise a first target measurement unit and a second target measurement unit,
   the first target measurement unit is configured to aspirate the identified second sample from a sample container containing the second sample and measure the aspirated second sample for the one or more preset measurement items, and
   the second target measurement unit is configured to aspirate the identified second sample from the sample container and measure the aspirated second sample for the one or more preset measurement items.

5. The sample analysis system (100) of any one of claims 1 - 4, wherein the computer is configured to:

   identify a plurality of second samples from the plurality of first samples; and
   for each of the identified second samples, store the plurality of second measurement results in the storage device for use in the inter-instrument difference evaluation.

6. The sample analysis system (100) of claim 5, wherein the identification information of each of the identified second samples is different from each other.

7. The sample analysis system (100) of any one of claims 1 - 6, wherein the computer is configured to determine the timing based on a preset timing for the inter-instrument difference evaluation and/or a reading of a preset identification information by the identification information reader.

8. The sample analysis system (100) of any one of claims 1 - 7, wherein the computer is configured to identify, as the second sample, the first sample that meets a preset sample condition.

9. The sample analysis system (100) of claim 8, wherein the sample condition is a condition set by an operator.

10. The sample analysis system (100) of claim 8 or 9, wherein the sample condition comprises at least one of: a condition on the first measurement result; and a condition on a quantity of the first sample.

11. The sample analysis system (100) of any one of claims 1 - 10, wherein the computer is configured to identify, as the second sample, the first sample that has been measured by at least one of the plurality of measurement units.

12. The sample analysis system (100) of any one of claims 1 - 11, wherein the computer is configured to identify, as the second sample, the first sample being associated with a preset identification information.

13. The sample analysis system (100) of claim 12, wherein the preset identification information comprises identification information uniquely identifying a sample for the inter-instrument difference evaluation.

14. The sample analysis system (100) of any one of claims 1 - 13, wherein the computer is configured to obtain the second measurement order based on identifying the second sample, and transmit the second measurement order to each of the plurality of target measurement units.

15. The sample analysis system (100) of any one of claims 1 - 14, wherein the one or more preset measurement items are set by an operator.

16. The sample analysis system (100) of any one of claims 1 - 15, further comprising a transport device configured to transport a sample container containing the identified second sample to each of the plurality of target measurement units.

17. The sample analysis system (100) of any one of claims 1 - 16, wherein the computer is configured to identify the second sample from the plurality of first samples whose identification information has been read by the identification information reader.

18. The sample analysis system (100) of any one of claims 1 - 17, wherein the computer is configured to identify the plurality of target measurement units from the plurality of measurement units.

19. The sample analysis system (100) of claim 18, wherein the computer is configured to identify the plurality of target measurement units designated by an operator.

20. The sample analysis system (100) of any one of claims 1 - 19, wherein the plurality of measurement units comprise a measurement unit serving as a master device which is used for obtaining a measurement result serving as a reference for the inter-instrument difference evaluation.

21. The sample analysis system (100) of claim 20, wherein the master device is set from the plurality of measurement units.

22. The sample analysis system (100) of any one of claims 1 - 21, wherein each of the target measurement units is a blood cell counting unit configured to count blood cells in a blood sample.

23. The sample analysis system (100) of any one of claims 1 - 22, wherein the computer is configured to generate evaluation information for the inter-instrument difference evaluation based on the plurality of second measurement results stored in the storage device.

24. A computer program product (115) comprising instructions which cause a computer (110) to carry out operations for inter-instrument difference evaluation, wherein the computer is in communication with:

a plurality of measurement units (160), each configured to measure a sample to obtain a measurement result based on a measurement order including one or more measurement items; and
an identification information reader configured to read identification information of each of a plurality of first samples to be measured for diagnosis of a test subject by at least one of the plurality of measurement units, wherein the first sample is measured by at least one of the plurality of measurement units based on a first measurement order for the diagnosis of the test subject, and a first measurement result of the first sample obtained from the at least one of the plurality of measurement units is stored, for use in the diagnosis of the test subject, in a storage device in association with the identification information of the first sample, and a plurality of target measurement units designated from the plurality of measurement units are subject for inter-instrument difference evaluation, wherein the operations for the inter-instrument difference evaluation comprise:

identifying at least one of the plurality of first samples as a second sample used for the measurement for the inter-instrument difference evaluation, based on a timing to conduct the inter-instrument difference evaluation, wherein the timing is automatically determined by the computer; and

controlling each of the plurality of target measurement units to measure the identified second sample based on a second measurement order including one or more preset measurement items for the inter-instrument difference evaluation, and storing a plurality of second measurement results of the second sample obtained from the plurality of target measurement units, for use in the inter-instrument difference evaluation, in the storage device in association with the identification information of the second sample.

Sample analysis system

Computer

110

100

150 150 150

Analyzer 1 Analyzer 2 Analyzer 3

**Fig. 1**

110

Computer

Processor 111    Memory 112    120

Bus

114

Storage device 116

Program    Database

115    Measurement results

117

Interface

Display    Operation device

118    119

Communication device

113

Analyzer

150

**Fig. 2**

Analyzer
150

160  180

Measurement unit

Data analysis unit

**Fig. 3**

**Fig. 4**

Start

Step S1

Identify a target sample for measurement for evaluation of inter-instrument difference

Step S2

Measure the target sample by each of target measurement units for measurement item for evaluation of inter-instrument difference

Step S3

Store multiple measurement results of the target sample obtained by the target measurement units

End

**Fig. 5A**

Samples for diagnosis of test subjects

160

Measurement units

Target sample

160

Measurement
unit M1

160

Measurement
unit M2

160

Measurement
unit M3

Measurement items:
- Item a
- Item b
- Item c

Measurement items:
- Item a
- Item b
- Item c

Measurement items:
- Item a
- Item b
- Item c

**Fig. 5B**

## Set-up for Inter-instrument difference evaluation

| Target measurement units | Target sample(s) | Measurement item(s) |
|---|---|---|

**Instrument ID**

| ☑ | M1 |
|---|---|
| ☑ | M2 |
| ☑ | M3 |

**Sample ID**

| 11 |
|---|
| . . . |
| . . . |
| . . . |

[ Add ] [ Delete ]

**Items**

| WBC |
|---|
| RBC |
| HGB |
| PLT |
| . . . |

[ Add ] [ Delete ]

[ OK ] [ Cancel ]

**Fig. 5C**

A set of measurement results
of the target sample

| Sample ID | WBC | · · | PLT | Instrument ID | Date/Time |
|---|---|---|---|---|---|
| 11 | · | · | · | M1 | 2024-03-13 13:30:00 |
| 11 | · | · | · | M2 | 2024-03-13 13:31:00 |
| 11 | · | · | · | M3 | 2024-03-13 13:32:00 |
| 12 | · | · | · | M1 | 2024-03-13 13:33:00 |
| 13 | · | · | · | M1 | 2024-03-13 13:34:00 |
| · | · | · | · | · | · |

Fig. 6A

Start

Step S4

Search for measurement results of the target
sample from the storage device

Step S5

Generate evaluation information for the
evaluation of inter-instrument difference based
on the measurement results

End

**Fig. 6B**

1st set of measurement results
of the target sample

| Sample ID | WBC | · · | PLT | Instrument ID | Date/Time |
|-----------|-----|-----|-----|---------------|-----------|
| 11 | · | · | · | M1 | 2024-03-13 13:30:00 |
| 11 | · | · | · | M2 | 2024-03-13 13:31:00 |
| 11 | · | · | · | M3 | 2024-03-13 13:32:00 |
| 12 | · | · | · | M1 | 2024-03-13 13:33:00 |
| 12 | · | · | · | M2 | 2024-03-13 13:34:00 |
| 12 | · | · | · | M3 | 2024-03-13 13:35:00 |
| 13 | · | · | · | M1 | 2024-03-13 13:36:00 |
| · | · | · | · | · | · |

2nd set of measurement results
of the target sample

Fig. 6C

Sample ID: 11

| Parameter | Unit | Allowable range | Standard value | M1 | | M2 | | M3 | |
|-----------|------|-----------------|----------------|-------|------|-------|------|-------|------|
| | | | | value | dev. | value | dev. | value | dev. |
| WBC | tsd/µL | 4% | 6.7 | 6.4 | -0.3 | 7.2 | 0.5 | 6.6 | -0.1 |
| PLT | tsd/µL | 3% | 80 | 80 | 0 | 82 | 2 | 78 | -2 |
| HGB | g/dL | 5% | 6.4 | 6.6 | 0.2 | 6.2 | -0.2 | 6.5 | 0.1 |

**Fig. 7**

A set of measurement results
of the target sample

| Sample ID | Inter-instrument diff evaluation flag | WBC | · · | PLT | Instrument ID | Date/Time |
|---|---|---|---|---|---|---|
| 11 | 1 | · | · | · | M1 | 2024-03-13 13:30:00 |
| 11 | 1 | · | · | · | M2 | 2024-03-13 13:31:00 |
| 11 | 1 | · | · | · | M3 | 2024-03-13 13:32:00 |
| 11 | 0 | · | · | · | M1 | 2024-03-13 13:33:00 |
| 12 | 0 | · | · | · | M2 | 2024-03-13 13:34:00 |
| · | · | · | · | · | · | · |

**Fig. 8**

A set of measurement results
of the target sample

| Sample ID | Re-exam flag | WBC | · | · | PLT | Instrument ID | Date/Time |
|-----------|--------------|-----|---|---|-----|---------------|-----------|
| 11 | 0 | · | · | · | M1 | 2024-03-13 13:30:00 |
| 11 | 0 | · | · | · | M2 | 2024-03-13 13:31:00 |
| 11 | 0 | · | · | · | M3 | 2024-03-13 13:32:00 |
| 12 | 0 | · | · | · | M1 | 2024-03-13 13:33:00 |
| 12 | 1 | · | · | · | M2 | 2024-03-13 13:34:00 |
| · | · | · | · | · | · | · |

Fig. 9

Computer

Start

Search for the set of measurement results of the target
sample from the storage device — Step S11

Meet condition ? — Step S12

NO

YES

Generate evaluation information for the inter-
instrument difference evaluation — Step S13

Other sets of
measurement results? — Step S14

YES

NO

End

**Fig. 10**

180'

160

Data analysis unit 183'

Measurement unit 2

160

Storage device 183b

Measurement unit 3

Program

Database

183a

Measurement results

160

Measurement unit 1

Fig. 11

Sample analysis system — 200

Fig. 12

Computer

Start

| Identify target sample | Step S21 |

| Transport target sample to target measurement units and measure target sample | Step S22 |

| Generate evaluation information | Step S23 |

End

Fig. 13

| Interval setting |
|---|

Time period

○ Day      Every [    ] days

○ Week      Every [    ] weeks

               ☐ Sun.    ☐ Mon.    ☐ Tue.    ☐ Wed.

               ☐ Thu.    ☐ Fri.      ☐ Sat.

○ Month      Every [    ] Month(s) [    ] day

○ Year      Every [    ] Year(s)    Date [    ] ▦

Fig. 14

| Number of target samples |
|---|
| N = ☐ |

Fig. 15

Computer

( Start )

↓

| Identify N target samples | Step S21A |

↓

| Transport N target samples to target measurement units and measure N target samples | Step S22A |

↓

| Generate evaluation information | Step S23A |

↓

( End )

Fig. 16

WBC

|  | **M1** | **M2** | **M3** |
|---|---|---|---|
| Date | 2024/3/13 | 2024/3/13 | 2024/3/13 |
| Time | 08:35:16:AM | 08:35:17:AM | 08:35:18:AM |
| Precision rate | 89% ! | 92% | 100% |
| Regression coefficient | 0.95 | 0.97 | 1.06 ! |
| N | 20 | 20 | 20 |

PLT

|  | **M1** | **M2** | **M3** |
|---|---|---|---|
| . | . | . | . |
| . | . | . | . |

Fig. 17

Sample analysis system

**Fig. 18**

Target sample 1

Target sample 2

Measurement
unit 1

350

350′

Measurement
unit 3

350

Master
device

Measurement
unit 2

350

Target sample 3

**Fig. 19**

**Fig. 20**

```
                        ┌─────────────┐
                        │    Start    │
                        └──────┬──────┘
                               │          Step S101
                        ┌──────▼──────────────┐
                        │   Read sample ID    │
                        └──────┬──────────────┘
                               │          Step S102
                          ╱────▼────╲
                         ╱ Unique    ╲      NO
                        ╱  identifier  ╲─────────────┐
                        ╲ in sample ID?╱             │
                         ╲────┬───────╱              │
                         YES  │  Step S103           │  Step S104
        ┌─────────────────────▼─────────┐  ┌─────────▼──────────────────┐
        │ Transport sample to target    │  │ Transport sample to at least│
        │ measurement units and         │  │ one of measurement units and│
        │ measure the sample according  │  │ measure the sample according│
        │ to measurement order for inter-│ │ to measurement order for    │
        │ instrument difference evaluation│ │ diagnosis of patient       │
        └─────────────────┬──────────────┘  └────────────┬──────────────┘
                          │◄──────────────────────────────┘
                          │            Step S105
        ┌─────────────────▼──────────────┐
        │ Store measurement results with │
        │ sample ID and Instrument ID    │
        └─────────────────┬──────────────┘
                          │
                   ┌──────▼──────┐
                   │     End     │
                   └─────────────┘
```

**Fig. 21**

47

A set of measurement
results of the target sample

| Sample ID | WBC | · · | PLT | Instrument ID | Date/Time |
|-----------|-----|-----|-----|---------------|-----------|
| 11 | · | · | · | M1 | 2024-03-13 13:20:00 |
| PSC11 | · | · | · | M1 | 2024-03-13 13:30:00 |
| PSC11 | · | · | · | M2 | 2024-03-13 13:31:00 |
| PSC11 | · | · | · | M3 | 2024-03-13 13:32:00 |
| 12 | · | · | · | M2 | 2024-03-13 13:34:00 |
| · | · | · | · | · | · |

**Fig. 22**

Special sample rack

Label including special rack ID

**Fig. 23**

Start

Step S201

Read sample ID and rack ID

Step S202

Unique identifier
in rack ID?

NO

YES Step S203

Transport sample rack to target
measurement units and
measure each sample in the
sample rack according to
measurement order for inter-
instrument difference evaluation

Step S204

Transport sample rack to at least
one of measurement units and
measure each sample in the
sample rack according to
measurement order for each
sample

Store measurement results with
sample ID, rack ID and
Instrument ID

Step S205

End

**Fig. 24**

A set of measurement
results of the target sample

| Rack ID | Sample ID | WBC | · · | PLT | Instrument ID | Date/Time |
|---------|-----------|-----|-----|-----|---------------|-----------|
| PSCR | 11 | · | · | · | M1 | 2024-03-13 13:30:00 |
| PSCR | 11 | · | · | · | M2 | 2024-03-13 13:31:00 |
| PSCR | 11 | · | · | · | M3 | 2024-03-13 13:32:00 |
| R2 | 12 | · | · | · | M1 | 2024-03-13 13:33:00 |
| R3 | 13 | · | · | · | M2 | 2024-03-13 13:34:00 |
| · | · | · | · | · | · | · |

Fig. 25

Fig. 26

**Fig. 27**

Storage device

Program

615

616

Database

Measurement results

Selection criteria

614

**Fig. 28**

Criteria table

| Criteria ID | Criteria |
|---|---|
| 1 | WBC_6 < M < 10 **and** PLT_M > 100 **and** HGB_M < 8 |
| 2 | WBC_M < 6 **and** PLT_M < 100 **and** HGB_M > 8 |
| 3 | Message_ "NRBC present" |
| 4 | Flag_ "R" |

**Fig. 29**

| Parameter | Rule | Value | Unit |
|---|---|---|---|
| WBC ⌄ | < ⌄ | 10 ⌄ | tsd/µL ⌄ |
| WBC ⌄ | > ⌄ | 6 ⌄ | tsd/µL ⌄ |
| PLT ⌄ | > ⌄ | 100 ⌄ | tsd/µL ⌄ |
| HGB ⌄ | < ⌄ | 8 ⌄ | g/dL ⌄ |

Fig. 30

| Message | On/Off |
|---|---|
| NRBC present ⌄ | On ⌄ |
| Left shift? ⌄ | Off ⌄ |

Fig. 31

| Alert flag | On/Off |
|---|---|
| R ⌄ | On ⌄ |
| H ⌄ | Off ⌄ |

Fig. 32

Target samples

| Sample ID | Criteria ID |
|---|---|
| 11 | Criteria 1 |
| 12 | Criteria 1 |
| 13 | Criteria 2 |

Fig. 33

Computer

Start

Report instruction with
target time period?
Step S51

NO

YES

Search for at least one set of measurement
results of a target sample within the
designated time period
Step S52

Output report
Step S53

End

**Fig. 34**

## Report for inter-instrument difference evaluation

- Target time period: 2024/3/13

- Target measurement units: M1, M2, M3

Overall evaluation

| Item | Determination condition | Determination result |
|------|------------------------|---------------------|
| WBC | Allowable range: +-4%, Precision rate: $90 \leqq p$ | NG |
| PLT | Allowable range: +-3%, Precision rate: $90 \leqq p$ | OK |
| HGB | Allowable range: +-5%, Precision rate: $90 \leqq p$ | OK |
| . | . | . |

Details

| | M1 | M2 | M3 |
|------|------|------|------|
| WBC | p=89 | p=92 | p=100 |
| PLT | p=90 | p=91 | p=93 |
| . | . | . | . |

**Fig. 35**

Europäisches
Patentamt
European
Patent Office
Office européen
des brevets

**EUROPEAN SEARCH REPORT**

Application Number

EP 24 22 1535

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/316461 A1 (FUJITA KYOZO [JP]) 28 November 2013 (2013-11-28) * The whole document, in particular: Paragraphs [0006], [0035], [0043] - [0047], [0050], [0051], [0074] - [0077], [0083] - [0085], [0114] - [0136], [0160] - [0162]; Figures 1, 3, 4, 6 - 8, 9A, 9B, 9C, 9D, 10, 16. * | 1-24 | INV. G16H10/40 G01N35/00 G16H40/40 |
| A | ALBERT RABINOVITCH ET AL: "Standard H26-A2: Validation, Verification and Quality of Automated Hematology Analyzers; Approved Standard- second Edition", STANDARD CLINICAL AND LABORATORY STANDARDS INSTITUTE, CLINICAL AND LABORATORY STANDARDS INSTITUTE, US, PAGE(S) 1 - 104 , 25 June 2010 (2010-06-25), XP009541363, ISBN: 978-1-56238-728-0 Retrieved from the Internet: URL:https://clsi.org/standards/products/hematology/documents/h26/ * The whole document, in particular: Sections *5.6 Calibration and Quality Control* on Pages 21 - 24 and *7.5 Quantitative Quality Control* on Pages 48 - 55. * | 1-24 | |

TECHNICAL FIELDS SEARCHED (IPC)

G16H
G01N

The present search report has been drawn up for all claims

| Place of search | Date of completion of the search | Examiner |
|---|---|---|
| Munich | 27 May 2025 | Ruschke, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 1535

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2025

| Patent document cited in search report | Publication date | Patent family member(s) | Publication date |
|---|---|---|---|
| US 2013316461 A1 | 28-11-2013 | CN 103364572 A | 23-10-2013 |
| | | JP 2013210265 A | 10-10-2013 |
| | | US 2013316461 A1 | 28-11-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S. PARK et al.** Development and Validation of Effective Real-Time and Periodic Interinstrument Comparison Method for Automatic Hematology Analyzers. *Am J Clin Pathol*, December 2014, vol. 142, 777-787